# EUROPEAN PATENT APPLICATION

(11) **EP 4 050 343 A1**
(43) Date of publication of application: **31.08.2022**
(21) Application number: 22158224.0
(22) Date of filing: 23.02.2022
(51) Int. Cl.: G01N 35/00, G01N 15/14, G06F 16/28, G16B 45/00

(54) **DISPLAY METHOD**

(30) Priority: 26.02.2021 JP 2021030969
(71) Applicant: SYSMEX CORPORATION, Kobe-shi Hyogo 651-0073 (JP)
(72) Inventor: MASUDA, Yuji, Kobe-shi, Hyogo, 651-0073 (JP); KANEKO, Tetsuya, Kobe-shi, Hyogo, 651-0073 (JP)
(74) Representative: Hoffmann Eitle

(57) **Abstract**

Disclosed is a method for displaying a result of analysis performed on a specimen containing formed components. The method includes: obtaining, from each of the formed components, measurement values of at least three parameters; displaying a screen including a three-dimensional distribution chart in which the three parameters are used as axes and the formed components are represented by dots; and hiding specific dots on the three-dimensional distribution chart or changing a display format of the specific dots, according to an operation performed by a user.

## Description

### CROSS REFERENCE TO RELATED APPLICATIONS

This application claims priority from prior Japanese Patent Application No. 2021-030969, filed on February 26, 2021, entitled "DISPLAY METHOD AND SPECIMEN ANALYSIS DEVICE", the entire content of which is incorporated herein by reference.

### FIELD OF THE INVENTION

The present invention relates to a display method and a specimen analysis device.

### BACKGROUND

An analysis device for classifying and analyzing a specimen containing formed components for each formed component through flow cytometry, has been known. Japanese Laid-Open Patent Publication No. 2014-106059 describes that classified formed components are displayed on a screen by means of a three-dimensional distribution chart.

### SUMMARY OF THE INVENTION

In the three-dimensional distribution chart, of Japanese Laid-Open Patent Publication No. 2014-106059, in which dots corresponding to the classified formed components are displayed, groups of dots corresponding to different types of formed components may overlap each other, which makes it difficult to visually recognize distribution of a desired formed component.

An object of the present invention is to provide a display method and a specimen analysis device in which visibility of distribution of formed components in a three-dimensional distribution chart is improved.

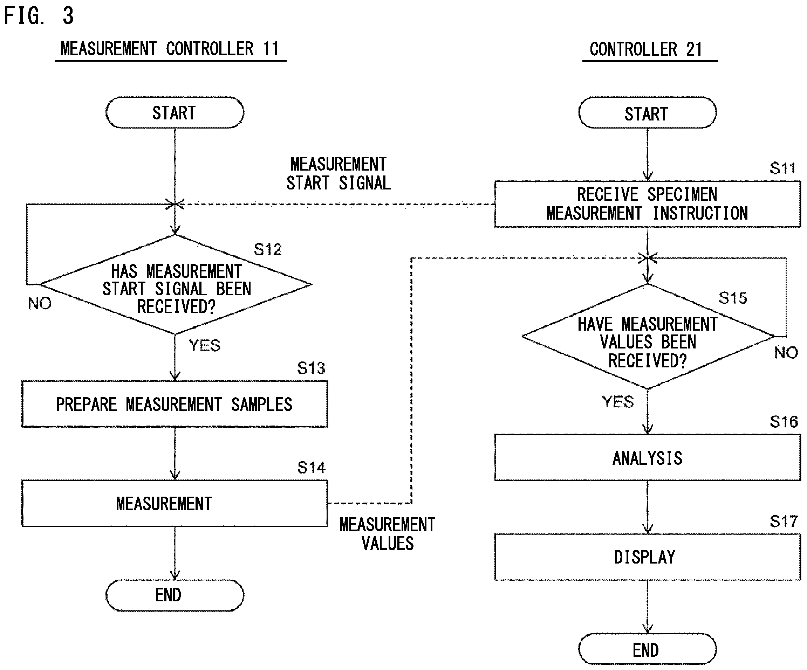

A display method according to the present invention, as shown in FIGS. 3, 7B, and 12, includes: a step (S14) of obtaining, from each of formed components, measurement values of at least three parameters; a step (S1741) of displaying a screen (300) including a three-dimensional distribution chart (310) in which the three parameters are used as axes (311 to 313) and the formed components are represented by dots; and a step (S1743) of hiding specific dots on the three-dimensional distribution chart (310) or changing a display format of the specific dots, according to an operation performed by a user.

According to the display method of the present invention, the user can hide dots, which are not required for analysis of a desired formed component, on the three-dimensional distribution chart. Alternatively, the user can display the dots, which are not required for analysis of the desired formed component, with the display format of the dots being changed on the three-dimensional distribution chart. Thus, the dots of the formed component desired by the user can be displayed on the three-dimensional distribution chart in an easily viewable manner, thereby improving visibility of distribution of the formed component on the three-dimensional distribution chart.

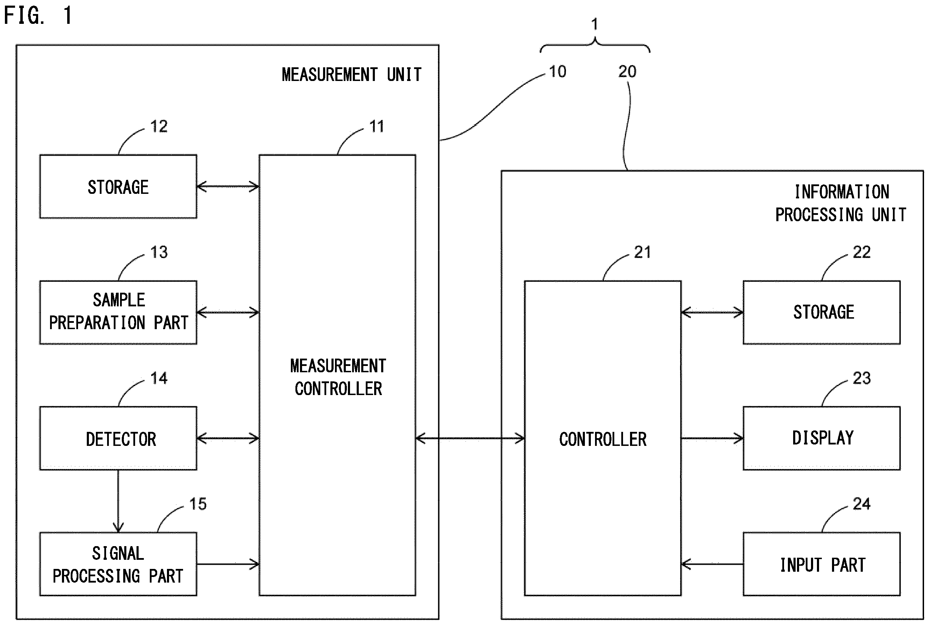

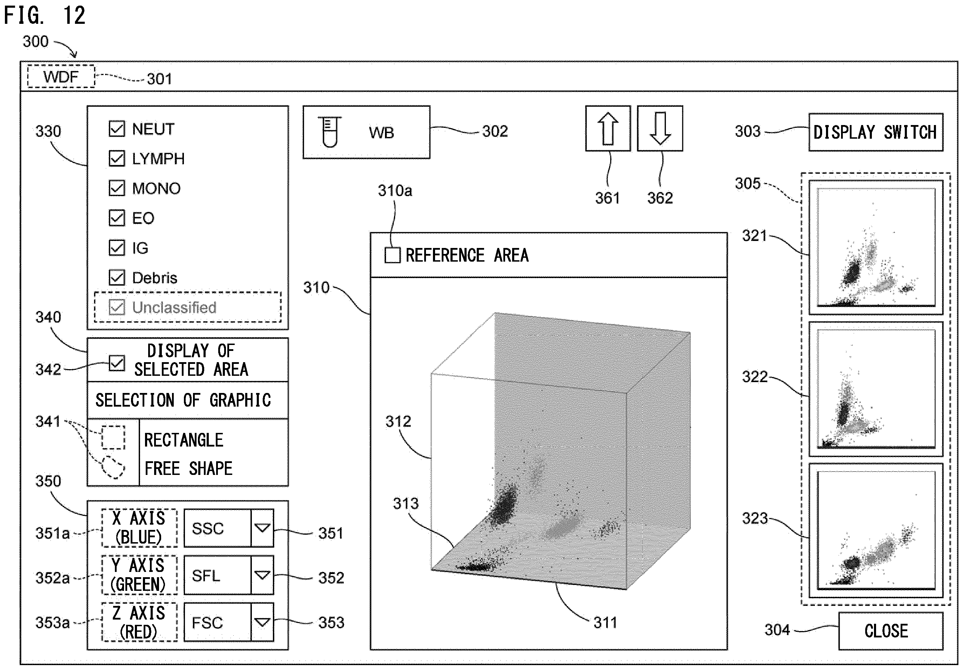

A specimen analysis device (1) according to the present invention, as shown in FIGS. 1 and 12, includes: a detector (14) configured to obtain a detection signal from each of formed components; a signal processing part (15) configured to obtain measurement values of at least three parameters from the detection signal; a display (23) configured to display a screen (300) including a three-dimensional distribution chart (310) in which the three parameters are used as axes (311 to 313) and the formed components are represented by dots; and a controller (21) configured to control, according to an operation performed by a user, the display (23) to hide specific dots on the three-dimensional distribution chart (310) or change a display format of the specific dots.

According to the specimen analysis device of the present invention, the user can hide dots, which are not required for analysis of a desired formed component, on the three-dimensional distribution chart. Alternatively, the user can display the dots, which are not required for analysis of the desired formed component, with the display format of the dots being changed on the three-dimensional distribution chart. Thus, the dots of the formed component desired by the user can be displayed on the three-dimensional distribution chart in an easily viewable manner, thereby improving visibility of distribution of the formed component on the three-dimensional distribution chart.

A display method according to the present invention includes: a step (S14) of obtaining measurement values from each of formed components; a step (S16) of obtaining a data set including at least three parameters based on the measurement values, and an attribute of the formed component; a step (S1741) of displaying a screen (300) including a three-dimensional distribution chart (310) in which the formed component is represented by dots, on the basis of the data set; and a step (S1743) of displaying/hiding the dots on the three-dimensional distribution chart (310) or changing a display format of the dots, on the basis of the attribute.

According to the display method of the present invention, dots, which are not required for analysis of a predetermined formed component, are displayed with presence/absence of display of the dots being changed on the three-dimensional distribution chart. Alternatively, the dots, which are not required for analysis of the predetermined formed component, are displayed with the display format of the dots being changed on the three-dimensional distribution chart. Thus, the dots of the predetermined formed component can be displayed on the three-dimensional distribution chart in an easily viewable manner, thereby improving visibility of distribution of the formed component on the three-dimensional distribution chart.

According to the present invention, visibility of distribution of formed components on a three-dimensional distribution chart can be improved.

### BRIEF DESCRIPTION OF THE DRAWINGS

FIG. 1 is a block diagram showing a configuration of a specimen analysis device according to Embodiment 1;
FIG. 2 is a plan view schematically showing a configuration of a detector according to Embodiment 1;
FIG. 3 is a flowchart showing a process performed by the specimen analysis device according to Embodiment 1;
FIG. 4 is a flowchart showing a formed component classification process according to Embodiment 1;
FIG. 5 schematically shows a configuration of a database having, stored therein, classification indicating the types of formed components classified by a controller, and measurement values obtained for each formed component, according to Embodiment 1;
FIG. 6 schematically shows a table having, stored therein, values regarding display/hiding for each classification, according to Embodiment 1;
FIG. 7A is a flowchart showing a specific process at S 17 in FIG. 3 according to Embodiment 1;
FIG. 7B is a flowchart showing a specific process at S 174 in FIG. 7A according to Embodiment 1;
FIG. 8 is a flowchart showing a specific process at S 1741 in FIG. 7B according to Embodiment 1;
FIG. 9 schematically shows a screen displayed on a display according to Embodiment 1;
FIG. 10 schematically shows a screen displayed on the display according to Embodiment 1;
FIG. 11 schematically shows a screen displayed on the display according to Embodiment 1;
FIG. 12 schematically shows a configuration of a screen including a three-dimensional distribution chart displayed on the display according to Embodiment 1;
FIGS. 13A and 13B show three-dimensional distribution charts in which display of reference areas is OFF and ON, respectively, according to Embodiment 1;
FIG. 14 schematically shows a configuration of a screen in which surface plot charts are displayed by an operation performed on a display switch button according to Embodiment 1;
FIG. 15 shows parameters of axes settable on a three-dimensional distribution chart according to measurement channels, and default parameters on three axes of the three-dimensional distribution chart, according to Embodiment 1;
FIGS. 16A to 16D show three-dimensional distribution charts according to Display Example 1 of Embodiment 1;
FIG. 17 schematically shows a screen including a three-dimensional distribution chart according to Display Example 2 of Embodiment 1;
FIG. 18 schematically shows a screen including a three-dimensional distribution chart according to Display Example 2 of Embodiment 1;
FIG. 19 schematically shows a screen including a three-dimensional distribution chart according to Display Example 2 of Embodiment 1;
FIG. 20 schematically shows a screen including a three-dimensional distribution chart according to Display Example 2 of Embodiment 1;
FIG. 21 schematically shows a screen including a three-dimensional distribution chart according to Display Example 2 of Embodiment 1;
FIG. 22 schematically shows a screen including a three-dimensional distribution chart according to Display Example 2 of Embodiment 1;
FIG. 23 schematically shows a screen including a three-dimensional distribution chart according to Display Example 2 of Embodiment 1;
FIG. 24A shows a two-dimensional distribution chart according to Display Example 3 of Embodiment 1;
FIG. 24B shows a three-dimensional distribution chart according to Display Example 3 of Embodiment 1;
FIGS. 25A and 25B show three-dimensional distribution charts according to Display Example 4 of Embodiment 1;
FIGS. 26A and 26C show two-dimensional distribution charts according to Display Example 5 of Embodiment 1;
FIGS. 26B and 26D show three-dimensional distribution charts according to Display Example 5 of Embodiment 1;
FIGS. 27A and 27B illustrate the colors of planes of a rectangular parallelepiped shape of a three-dimensional distribution chart, according to Embodiment 1;
FIGS. 28A and 28B show three-dimensional distribution charts according to Modification 1 of Embodiment 1;
FIGS. 29A and 29B show three-dimensional distribution charts according to Modification 2-1 of Embodiment 1;
FIGS. 30A and 30B show three-dimensional distribution charts according to Modification 2-2 of Embodiment 1;
FIGS. 31A and 31B schematically show two-dimensional distribution charts according to a display example of Embodiment 2;
FIG. 32 schematically shows a two-dimensional distribution chart according to the display example of Embodiment 2;
FIGS. 33A and 33B schematically show three-dimensional distribution charts according to the display example of Embodiment 2; and
FIGS. 34A and 34B schematically show three-dimensional distribution charts according to the display example of Embodiment 2.

### DETAILED DESCRIPTION OF THE PREFERRED EMBODIMENTS

The following embodiments are obtained by applying the present disclosure to a display method for displaying a result of analysis performed on a specimen containing formed components, and a specimen analysis device for analyzing a specimen containing formed components.

### <Embodiment 1>

FIG. 1 is a block diagram showing the configuration of a specimen analysis device 1.

The specimen analysis device 1 of Embodiment 1 analyzes blood and body fluid as specimens. The specimen analysis device 1 includes a measurement unit 10 and an information processing unit 20.

The measurement unit 10 includes a measurement controller 11, a storage 12, a sample preparation part 13, a detector 14, and a signal processing part 15.

The measurement controller 11 is implemented by a CPU, for example. The measurement controller 11 receives a signal outputted from each of the components of the measurement unit 10, and controls the components of the measurement unit 10. The measurement controller 11 communicates with the information processing unit 20. The storage 12 is implemented by, for example, a ROM, a RAM, a hard disk, or the like. The measurement controller 11 stores the signals from the detector 14 in the storage 12, and performs various processes based on a program stored in the storage 12.

The sample preparation part 13 aspirates a specimen from a container, and mixes the aspirated specimen with reagents to prepare various measurement samples. The specimen to be analyzed by the specimen analysis device 1 is whole blood when a measurement mode is any of a whole blood mode (WB), a low white blood cell mode (LW), and a pre-dilution mode (PD), and it is body fluid (cerebrospinal fluid, ascitic fluid, pleural fluid, synovial fluid, peritoneal dialysis effluent, etc.) when the measurement mode is a body fluid mode (BF). The sample preparation part 13 mixes the specimen with predetermined reagents to prepare a WNR measurement sample, a WDF measurement sample, a WPC measurement sample, a RET measurement sample, and a PLT-F measurement sample. The WNR measurement sample is a sample for measuring the number of all white blood cells and the number of nucleated red blood cells. The WDF measurement sample is a sample for classifying and counting white blood cells. The WPC measurement sample is a sample for detecting abnormal cells separately from mature white blood cells. The RET measurement sample is a sample for measuring the number of reticulocytes. The PLT-F measurement sample is a sample for optically measuring platelets.

The detector 14 is an optical detector that measures each measurement sample prepared by the sample preparation part 13, and outputs a detection signal. The detector 14 applies light to a flow cell in which the measurement sample flows, receives forward scattered light, side scattered light, and side fluorescence generated from a formed component in the measurement sample, and outputs a detection signal based on each light.

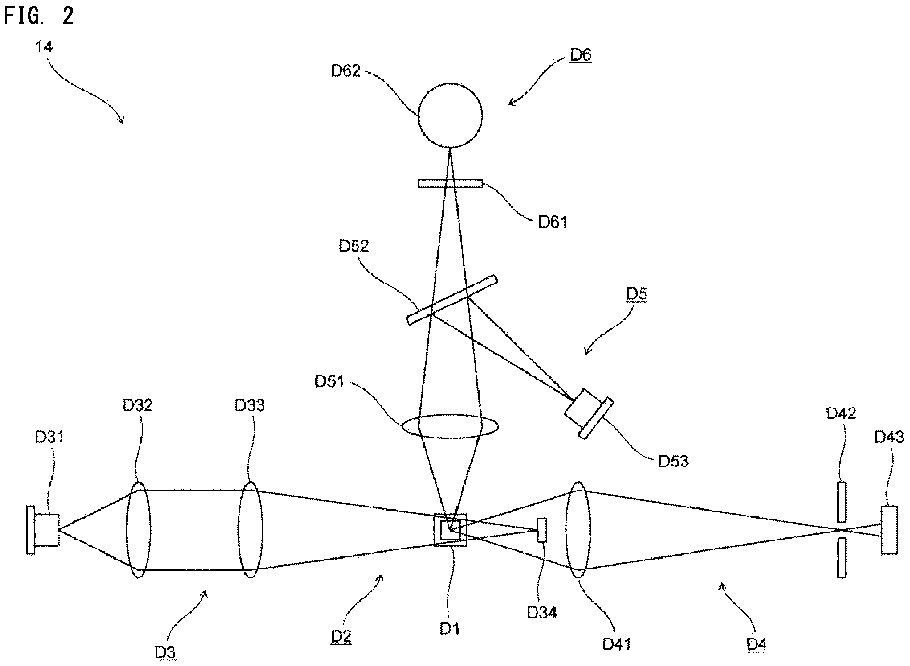

As shown in FIG. 2, the detector 14 includes a flow cell D1, a sheath flow system D2, a beam spot forming system D3, a forward scattered light receiving system D4, a side scattered light receiving system D5, and a fluorescence receiving system D6.

The sheath flow system D2 is configured to send the measurement sample surrounded by a sheath liquid into the flow cell D1 and generate a liquid flow in the flow cell D1. The beam spot forming system D3 is configured such that light emitted from a light source D31 passes through a collimator lens D32 and a condenser lens D33 and is applied to the flow cell D1. Thus, laser light is applied to the formed component in the measurement sample included in the liquid flow passing through the inside of the flow cell D1. The beam spot forming system D3 further includes a beam stopper D34. The light source D31 is a semiconductor laser light source, for example.

The forward scattered light receiving system D4 is configured to condense the forward scattered light by a forward condenser lens D41 and receive light, which has passed through a pinhole formed in a light blocking plate D42, by a photodiode D43 which is a first optical detector. The photodiode D43 outputs an analog detection signal having a waveform according to the intensity of the received forward scattered light. The side scattered light receiving system D5 is configured to condense the side scattered light by a side condenser lens D51, reflect a portion of the light by a dichroic mirror D52, and receive the reflected light by a photodiode D53 which is a second optical detector. The photodiode D53 outputs an analog detection signal having a waveform according to the intensity of the received side scattered light.

The fluorescence receiving system D6 is configured to: cause light (fluorescence), which has been transmitted through the dichroic mirror D52, of light generated on the side of the flow cell D1 to further pass through a spectral filter D61; and receive the fluorescence by an avalanche photodiode D62 which is a third optical detector. The avalanche photodiode D62 outputs an analog detection signal having a waveform according to the intensity of the received fluorescence.

The detector 14 may output another detection signal based on scattering of light, in addition to the forward scattered light and the side scattered light described above. The detection signals based on the forward scattered light and the side scattered light described above include detection signals defined by the scattering directions of the forward scattered light, the side scattered light, and the like. The forward scattered light is, when light is applied to a particle, light that is scattered forward with respect to the advancing direction of the applied light. The side scattered light is, when light is applied to a particle, light that is scattered laterally with respect to the advancing direction of the applied light. The angle of the lateral scattering may be any angle that is different from the scattering angle of the light detected as the forward scattered light. For example, when light generated at an angle of 0° to 10° with respect to the optical axial of the applied light is the forward scattered light to be detected, light generated at any angle greater than 10° may be detected as the side scattered light. In the example shown in FIG. 2, light generated in a direction of about 80° to 100° with respect to the optical axis is detected as the side scattered light. The side scattered light may include a plurality of different types of light according to the angle, such as low-angle scattered light and high-angle scattered light, for example. As another detection signal based on scattering of light, there is an axial light loss. The axial light loss is a detection signal obtained by quantifying a decrease, in the amount of received light at the light receiving side, due to scattering of light when a particle crosses laser light.

Measurement based on each measurement sample and performed by the detector 14 is called "measurement channel". More specifically, a measurement channel based on a WNR measurement sample is called "WNR channel", a measurement channel based on a WDF measurement sample is called "WDF channel", a measurement channel based on a WPC measurement sample is called "WPC channel", a measurement channel based on a RET measurement sample is called "RET channel", and a measurement channel based on a PLT-F measurement sample is called "PLT-F channel".

Referring back to FIG. 1, the signal processing part 15 is implemented by a circuit that generates a measurement value by performing A/D conversion on an analog detection signal outputted from the detector 14. The measurement controller 11 stores measurement values generated by the signal processing part 15 in the storage 12. When measurement for one specimen is finished, the measurement controller 11 transmits the measurement values stored in the storage 12 to the information processing unit 20.

The information processing unit 20 includes a controller 21, a storage 22, a display 23, and an input part 24.

The controller 21 is implemented by a CPU, for example. The controller 21 receives a signal outputted from each of the components of the information processing unit 20, and controls the components of the information processing unit 20. The controller 21 communicates with the measurement unit 10, and controls each of the components of the measurement unit 10 via the measurement controller 11. The controller 21 stores measurement values received from the measurement unit 10 in the storage 22.

Moreover, the controller 21 performs a predetermined control according to a control program stored in the storage 22. The control program implements a function of analyzing and counting formed components by using the measurement values.

The controller 21, by using the measurement values, classifies the formed components into a plurality of types, and obtains result values regarding measurement items such as a blood cell count.

The display 23 is implemented by a liquid crystal display, for example. The display 23 displays various screens in accordance with signals from the controller 21.

The input part 24 is implemented by a keyboard and a mouse. The user inputs an instruction to the controller 21 by performing, with the mouse of the input part 24, an operation such as click or double-click to a screen displayed on the display 23. The display 23 and the input part 24 may be implemented by a touch-panel type display. In this case, the user performs, instead of click or double-click, tap or double-tap to a display screen of the touch-panel type display.

FIG. 3 is a flowchart showing a process performed by the specimen analysis device 1.

In step S11, upon receiving a measurement instruction for a specimen, the controller 21 transmits a measurement start signal to the measurement controller 11.

In step S12, the measurement controller 11 receives the measurement start signal from the controller 21 (S12: YES), and advances the process to step S13.

In step S13, the measurement controller 11 controls the sample preparation part 13 so as to aspirate the specimen from a container and prepare a WNR measurement sample, a WDF measurement sample, a WPC measurement sample, a RET measurement sample, and a PLT-F measurement sample on the basis of the aspirated specimen. The measurement samples are selectively prepared on the basis of a measurement order for the specimen. In step S14, the measurement controller 11 measures, by the detector 14, the WNR measurement sample, the WDF measurement sample, the WPC measurement sample, the RET measurement sample, and the PLT-F measurement sample.

The signal processing part 15 generates measurement values on the basis of detection signals obtained in each measurement. Specifically, the signal processing part 15 obtains, for each measurement sample, measurement values of a plurality of parameters for each formed component, on the basis of the detection signals of the respective lights detected by the detector 14. For example, the measurement values of the parameters include: a forward scattered light intensity (FSC), a side scattered light intensity (SSC), and a side fluorescence intensity (SFL) which are obtained as waveform peak values; a forward scattered light width (FSCW) obtained as a waveform width; SFL_EXT that is SFL widely extended; and FSC LOG that is FSC converted to a log. The SFL EXT can be obtained by, for example, using a band width wider than a band width used for obtaining a normal side fluorescence intensity (SFL) when the signal processing part 15 converts an analog detection signal, which is outputted from the avalanche photodiode D62 as the third optical detector, into a digital signal. The measurement controller 11 transmits, to the controller 21, the measurement values of the plurality of parameters obtained for each formed component.

The measurement values of the parameters are not limited to the measurement values described above. For example, the measurement values of the parameters may include a side scattered light width (SSCW) and a side fluorescence width (SFLW) which are obtained as waveform widths, may include a forward scattered light area (FSCA), a side scattered light area (SSCA), and a side fluorescence area (SFLA) which are obtained as waveform areas, and may include measurement values obtained by widely extending FSC and SSC, and measurement values obtained by converting SSC and SFL to logs.

In step S15, the controller 21 receives the measurement values from the measurement controller 11 (S15: YES), and advances the process to step S16.

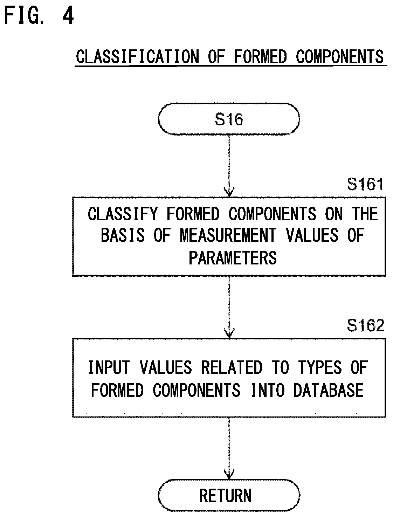

In step S16, the controller 21 classifies the formed components on the basis of the measurement values. The process in step S16 will be described in detail with reference to FIG. 4.

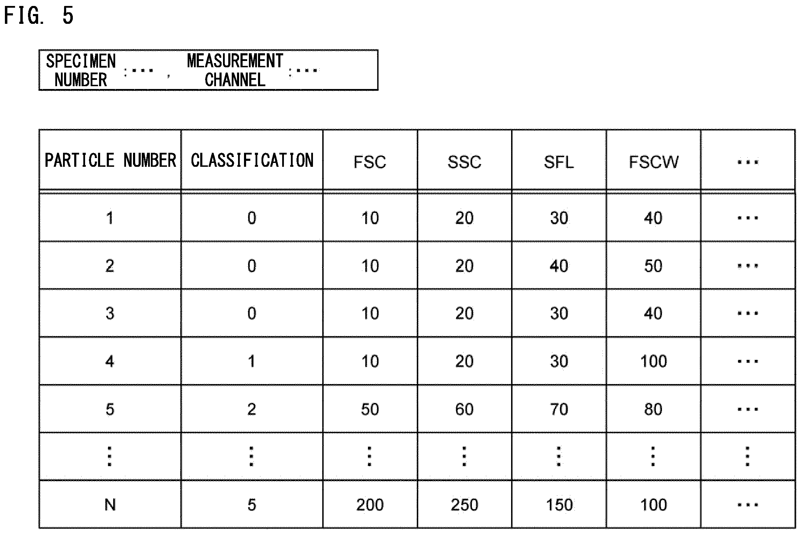

As shown in FIG. 4, in step S161, the controller 21 classifies the formed components (particles) into a plurality of types on the basis of the measurement values of the plurality of parameters obtained from the signal processing part 15. As a method for classifying the particles into the plurality of types by using the plurality of parameters, there is a method including: plotting the particles in a multidimensional coordinate space having the plurality of parameters as axes; classifying at least some particles into a plurality of clusters corresponding to the plurality of types; obtaining the belonging degree of each particle to the corresponding cluster on the basis of the distance between the particle and the position of the center of gravity of the cluster; and reclassifying the particles on the basis of the belonging degrees to classify the particles into the plurality of types. Such a classification method is disclosed in US Patent No. 5,555,198. US Patent No. 5,555,198 is incorporated herein by reference. In step S162, the controller 21 inputs, to a database shown in FIG. 5, classification corresponding to the formed components (particles) classified in step S161.

For example, the controller 21 classifies and counts nucleated red blood cells, basophils, white blood cells other than basophils, debris, and the like on the basis of the measurement values regarding the WNR channel. The controller 21 classifies and counts lymphocytes, monocytes, eosinophils, neutrophils, basophils, immature granulocytes, debris, and the like on the basis of the measurement values regarding the WDF channel. The controller 21 classifies and counts immature cells, abnormal cells, mature white blood cells, and the like on the basis of the measurement values regarding the WPC channel. The controller 21 classifies and counts reticulocytes, mature red blood cells, platelets, and the like on the basis of the measurement values regarding the RET channel. The controller 21 classifies and counts platelets, part of red blood cells, part of white blood cells, debris, and the like on the basis of the measurement values regarding the PLT-F channel.

The controller 21 stores, as measurement data (data set), in the storage 22, the result of classification of the formed components (particles), the result values of the measurement items, information on the specimen, the date and time of the measurement, and the like, in addition to the measurement values received from the measurement controller 11.

FIG. 5 schematically shows the configuration of the database having, stored therein, classification indicating the types of the formed components (particles) classified by the controller 21, and the measurement values obtained for the respective formed components.

In the storage 22 of the information processing unit 20, the classification and the measurement values as shown in FIG. 5 are stored as a database for each specimen number and each measurement channel. A particle number is a number with which a detected formed component (particle) can be identified.

Classification indicates a number corresponding to the type, of each formed component (particle), such as lymphocyte, monocyte, eosinophil, neutrophil, basophil, immature granulocyte, immature cell, abnormal cell, mature white blood cell, nucleated red blood cells, reticulocyte, mature red blood cell, platelet, debris, or unclassified. For example, classification values 0, 1, 2, 3, 4, and 5 correspond to neutrophil (NEUT), lymphocyte (LYMPH), monocyte (MONO), eosinophil (EO), immature granulocyte (IG), and Debris, respectively.

FSC, SSC, SFL, FSCW, etc., indicate the measurement values of the respective parameters. The controller 21 classifies the formed components (particles) into a plurality of types on the basis of the measurement values of the plurality of parameters. Classification indicates the values corresponding to the types of the classified formed components (particles). The controller 21 stores, in the storage 22, the classification indicating the types of the formed components (particles) and the measurement values as shown in FIG. 5. Then, as described later, the controller 21 renders dots according to the types of the formed components (particles) on coordinates corresponding to the measurement values of the parameters of the formed components (particles) on a two-dimensional distribution chart or a three-dimensional distribution chart.

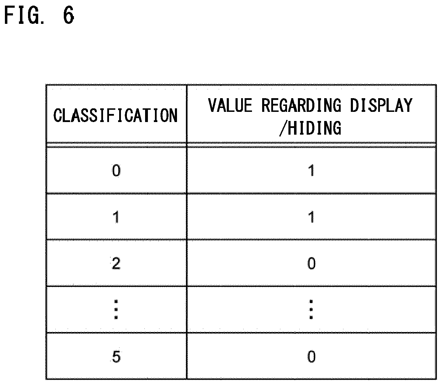

The controller 21 stores, in the storage 22, values regarding dot display/hiding shown in FIG. 6, in association with the classification, in accordance with setting of display/hiding of dots corresponding to formed components (particles) in a three-dimensional distribution chart 310 described later. In the example of FIG. 6, as for the values regarding display/hiding, "1" corresponds to display of a formed component (particle) and "0" corresponds to hiding of a formed component (particle).

Referring back to FIG. 3, in step S17, the controller 21 displays, on the display 23, a two-dimensional distribution chart, a three-dimensional distribution chart, a particle size distribution chart, a surface plot chart, and the like in response to an instruction of the user received through the input part 24.

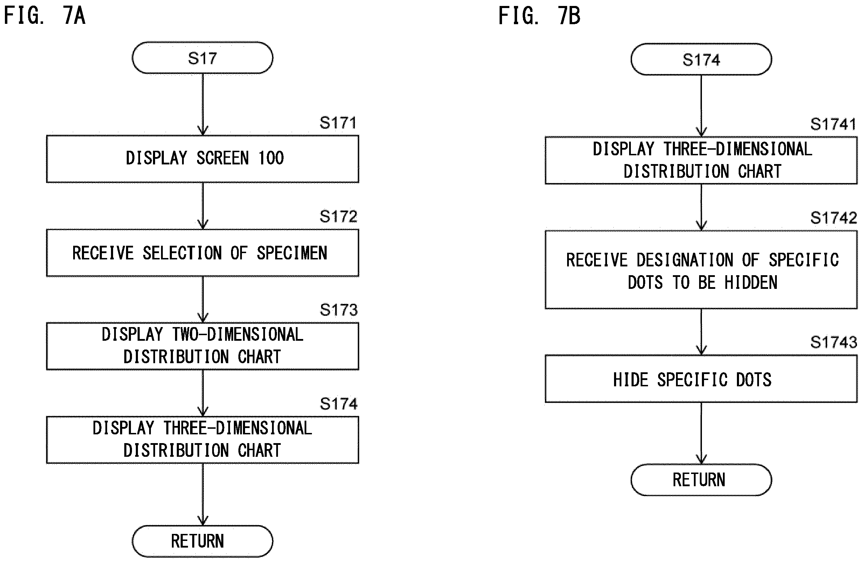

The process in step S17 will be described in detail with reference to FIG. 7A. In the following description, for example, a specimen in the whole blood mode (WB) is selected, and a three-dimensional distribution chart 310 regarding the WDF channel is displayed.

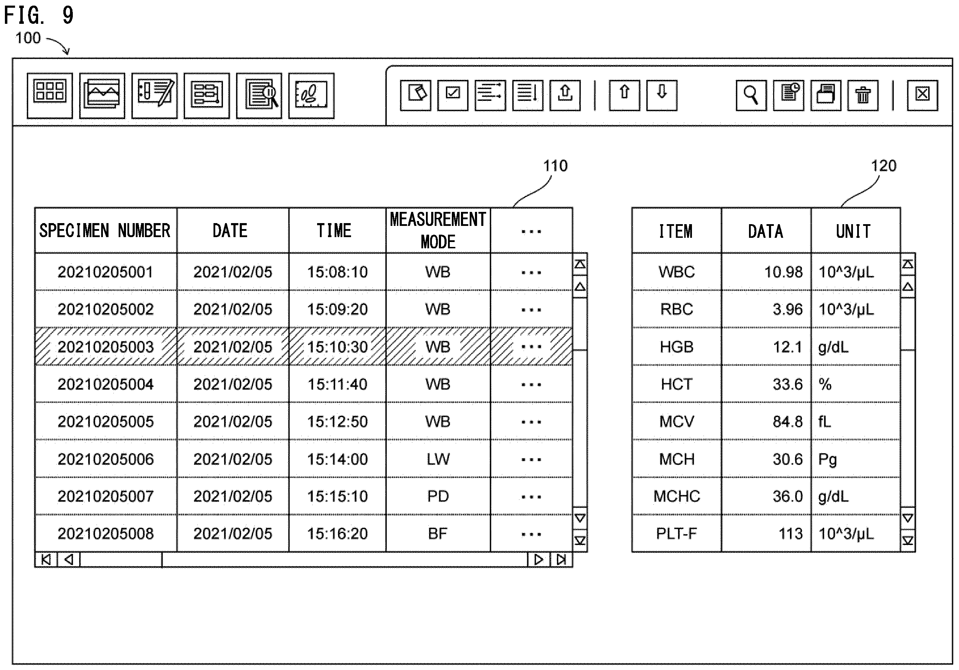

In step S171, the controller 21 displays, on the display 23, a screen 100 including specimen number, date, time, measurement mode, item (measurement item), data (result value), etc., as shown in FIG. 9, on the basis of the measurement values obtained in step S14 and the result values of the measurement items obtained in step S16. In step S172, the controller 21 receives selection of a specimen in the whole blood mode (WB) in response to an operation (e.g., an operation of double-clicking the row of the target specimen in a measurement data display area 110) performed by the user through the input part 24.

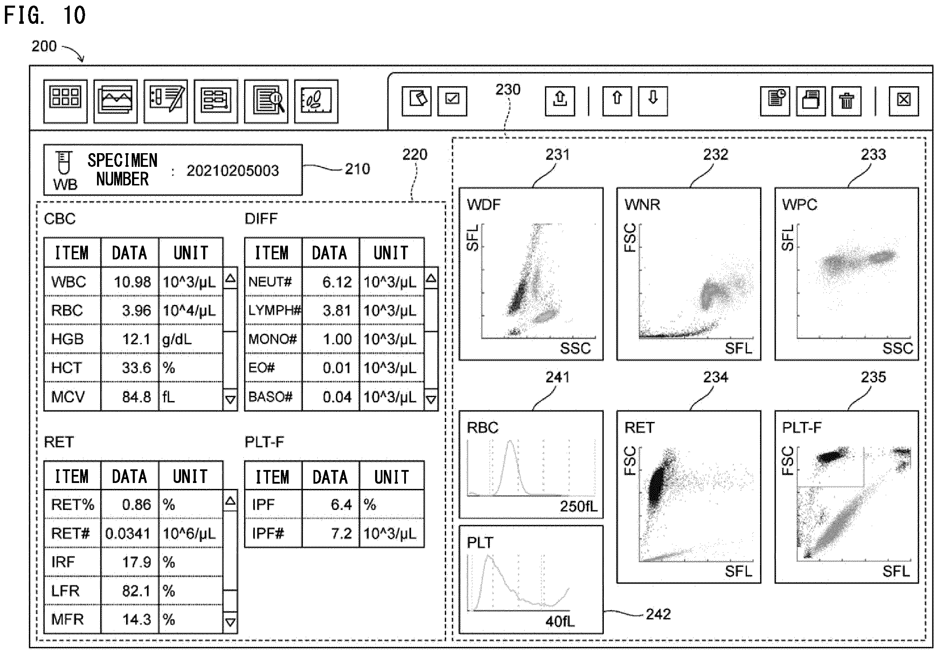

In step S173, the controller 21 displays, on the display 23, a two-dimensional distribution chart 231 (see FIG. 10) corresponding to the specimen in the whole blood mode (WB) having been selected. In step S174, the controller 21 displays, on the display 23, a three-dimensional distribution chart 310 (see FIG. 12) in response to an operation (e.g., an operation of double-clicking the two-dimensional distribution chart 231) performed by the user through the input part 24.

The process in step S174 will be described in detail with reference to FIG. 7B.

In step S1741, the controller 21 displays the three-dimensional distribution chart 310 (see FIG. 12) on the display 23, in response to an operation performed by the user through the input part 24. In step S1742, the controller 21 receives designation of specific dots corresponding to a formed component (particle) to be set to "hiding" on the three-dimensional distribution chart 310, in response to an operation (e.g., an operation to a type selection area 330 and a region selection area 340) performed by the user through the input part 24. The controller 21 updates the table shown in FIG. 6 on the basis of the received designation of "hiding".

In step S1743, the controller 21 performs display control for the screen of the display 23 such that the specific dots corresponding to the designated formed component (particle) are not displayed on the three-dimensional distribution chart 310. In step S1743, the controller 21 reads out the values, regarding dot display/hiding, which are stored in the storage 22 in association with the classification as shown in FIG. 6. The controller 21 displays the dots corresponding to the formed components (particles) whose values regarding dot display/hiding are "1", and hides the dots corresponding to the formed components (particles) whose values regarding dot display/hiding are "0".

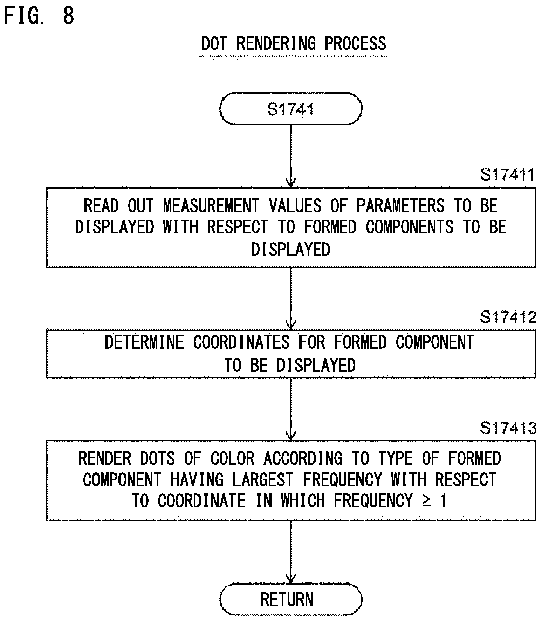

The process in step S1741 will be described in detail with reference to FIG. 8.

In step S17411, as for formed components to be displayed, the controller 21 reads out the measurement values of parameters to be displayed. In step S17411, the formed components to be displayed are all the formed components, the measurement values of which have been obtained from the optical detector, regarding the target specimen and the target measurement channel. As for the parameters to be displayed, parameters corresponding to coordinates (XYZ coordinate space) required for rendering of dots corresponding to formed components on a three-dimensional distribution chart described later are set as default parameters at initial setting. In step S17412, the controller 21 determines coordinates (XYZ coordinate space) of the formed components to be displayed, and counts the frequency at each coordinate.

In step S17413, as for a coordinate at which the frequency is 1 or higher, the controller 21 renders a dot of a color according to a type of formed component having the highest frequency, on the three-dimensional distribution chart.

The process in step S17413 will be described with reference to FIG. 5. The particle numbers 1, 3, and 4 have the same value for each of FSC, SSC, and SFL, and therefore, are plotted to the same coordinate in the three-dimensional coordinate space using FSC, SSC, and SFL as axes. The particle numbers 1 and 3 have the classification value "0" while the particle number 4 has the classification value "1". In this case, the frequency of the formed component corresponding to the classification value "0" on the above coordinate is 2 while the frequency of the formed component corresponding to the classification value "1" is 1. In this case, a dot of a color according to the classification value "0" corresponding to the formed component of the highest frequency is rendered. If two or more types of formed components of the same frequency are plotted to the same coordinate, a color of a dot is determined by using another parameter for determining superiority/inferiority. For example, among competing two or more types of formed components, a formed component, the center of gravity of a dot cluster of which is closest to the above coordinate, is given the color of the corresponding dot cluster.

The controller 21 receives an instruction of the user through the input part 24, and performs display control of a screen according to the received instruction, as described in FIG. 9 and subsequent figures.

Next, a screen displayed on the display 23 in step S17 in FIG. 3 will be described.

FIG. 9 schematically shows a screen 100 displayed on the display 23.

Upon receiving an operation, for displaying the screen 100, performed by the user on a menu screen, the controller 21 displays the screen 100 on the display 23 on the basis of the measurement data stored in the storage 22.

The screen 100 includes a measurement data display area 110 and a measurement item display area 120.

The measurement data display area 110 includes specimen number, date, time, measurement mode, etc., as items. The user can cause other specimens and other items to be displayed in the measurement data display area 110 by operating buttons and bars for scrolling the display area in the up-down direction and the left-right direction. Moreover, the user, by setting filter conditions, can change the arrangement order of the items in the measurement data display area 110 and cause specimens satisfying the conditions to be displayed.

The measurement item display area 120 includes item (measurement item), data (result value), and unit, as items. The user can cause other measurement items to be displayed in the measurement item display area 120 by operating buttons and bars for scrolling the display area in the up-down direction.

When the user clicks a row extending in the transverse direction of the measurement data display area 110, the clicked row is displayed in a different color as shown in FIG. 9, and the result values of the measurement items regarding the selected specimen are displayed in the measurement item display area 120. Moreover, when the user double-clicks a row in the measurement data display area 110, a screen 200 listing the result values, etc., regarding the specimen in the double-clicked row is displayed on the display 23.

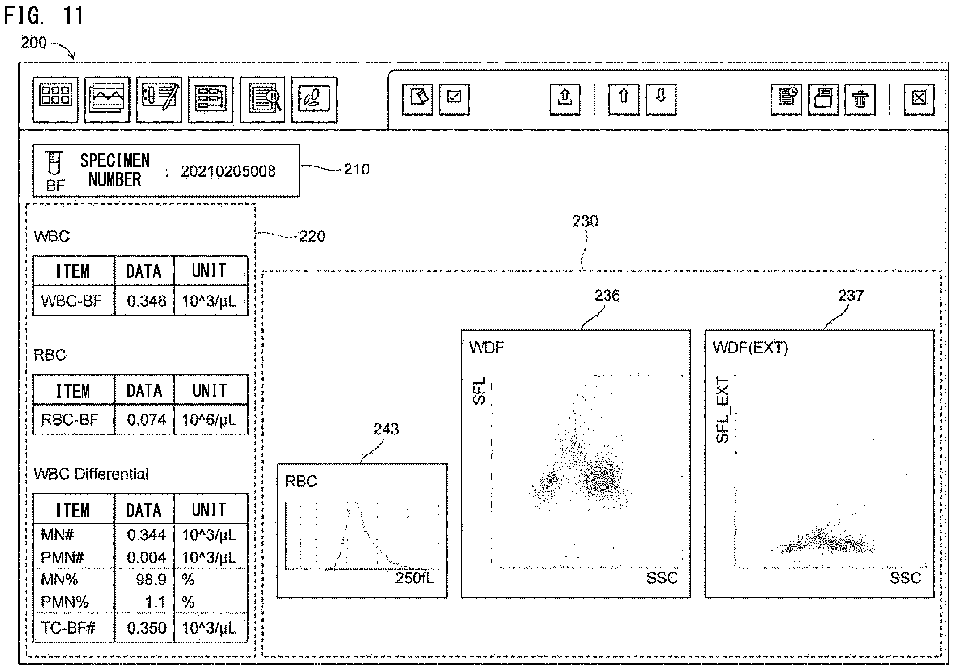

FIGS. 10 and 11 each schematically show the screen 200 displayed on the display 23.

The screen 200 shown in FIG. 10 is displayed when the specimen in the whole blood mode (WB) is double-clicked in FIG. 9. The screen 200 shown in FIG. 11 is displayed when the specimen in the body fluid mode (BF) is double-clicked in FIG. 9. Since screens to be displayed for the low white blood cell mode (LW) and the pre-dilution mode (PD) are substantially the same as the screen 200 shown in FIG. 10, description thereof is omitted.

As shown in FIG. 10, the screen 200 for the whole blood mode (WB) includes a specimen information area 210, a measurement item display area 220, and a graph display area 230.

The specimen information area 210 displays the specimen number of the specimen displayed on the screen 200, and the measurement mode of the specimen. In the example shown in FIG. 10, since the measurement mode is the whole blood mode (WB), the display contents of the measurement item display area 220 and the graph display area 230 conform to the whole blood mode.

The measurement item display area 220 displays the result values of the measurement items of the specimen displayed on the screen 200. The graph display area 230 displays two-dimensional distribution charts 231 to 235 and particle size distribution charts 241 and 242. In the two-dimensional distribution charts 231 to 235, dots corresponding to the formed components regarding the WDF channel, the WNR channel, the WPC channel, the RET channel, and the PLT-F channel are respectively displayed on the basis of the measurement values. The colors of the dots on the two-dimensional distribution charts 231 to 235 are different for each of the types of the formed components. The particle size distribution charts 241 and 242 are displayed on the basis of the measurement values regarding red blood cells and platelets, respectively.

As shown in FIG. 11, the screen 200 for the body fluid mode (BF) includes a specimen information area 210, a measurement item display area 220, and a graph display area 230, as in FIG. 10.

The specimen information area 210 displays the specimen number of the specimen displayed on the screen 200, and the measurement mode of the specimen. In the example shown in FIG. 11, since the measurement mode is the body fluid mode (BF), the display contents of the measurement item display area 220 and the graph display area 230 conform to the body fluid mode.

The measurement item display area 220 displays the result values of the measurement items of the specimen displayed on the screen 200. The graph display area 230 displays two-dimensional distribution charts 236 and 237 and a particle size distribution chart 243. In the two-dimensional distribution chart 236, as in the two-dimensional distribution chart 231 shown in FIG. 10, dots corresponding to the formed component regarding the WDF channel are displayed on the basis of the measurement values. The two-dimensional distribution chart 237 is a chart regarding the WDF channel like the two-dimensional distribution chart 236, but is different in the scale on the vertical axis (SFL) from the two-dimensional distribution chart 236. Specifically, an upper limit value of the vertical axis of the two-dimensional distribution chart 237 is larger than an upper limit value of the vertical axis of the two-dimensional distribution chart 236. The vertical axis of the two-dimensional distribution chart 237 is SFL EXT. The colors of the dots on the two-dimensional distribution charts 236 and 237 are different for each of the types of the formed components. The particle size distribution chart 243 is displayed on the basis of the measurement values regarding red blood cells.

When the user double-clicks any of the two-dimensional distribution charts 231 to 237 shown in FIGS. 10 and 11, a screen 300 displaying a three-dimensional distribution chart corresponding to the double-clicked two-dimensional distribution chart is displayed on the display 23.

FIG. 12 schematically shows the configuration of a screen 300, including a three-dimensional distribution chart, which is displayed on the display 23.

FIG. 12 shows an example of the screen 300 displayed when the two-dimensional distribution chart 231 corresponding to the WDF channel is double-clicked.

The screen 300 includes a measurement channel display area 301, a measurement mode display area 302, a display switch button 303, a close button 304, a three-dimensional distribution chart 310, two-dimensional distribution charts 321 to 323, a type selection area 330, a region selection area 340, an axis selection area 350, and specimen transition buttons 361 and 362.

The measurement channel display area 301 indicates a measurement channel, information of which is displayed on the screen 300. The measurement mode display area 302 indicates a measurement mode, information of which is displayed on the screen 300. FIG. 12 shows the state where the measurement channel is the WDF channel and the measurement mode is the whole blood mode (WB).

The three-dimensional distribution chart 310 is a three-dimensional distribution chart corresponding to a two-dimensional distribution chart that was designated by double-clicking when the screen 300 was displayed. Specifically, the three-dimensional distribution chart 310 is displayed such that another parameter is further added to the parameters (SSC and SFL in the case of the WDF channel) of the two axes of the original two-dimensional distribution chart. As for dots on the three-dimensional distribution chart 310, different types of dots are given different colors on the basis of the values of the classification values indicating the types of dots corresponding to the formed components (particles) shown in FIG. 5. When the user performs a drag operation on the display area in the three-dimensional distribution chart 310, the view angle of the three-dimensional distribution chart 310 is changed according to the drag operation. The view angle of the three-dimensional distribution chart 310 shown in FIG. 12 is a view angle at initial (default) setting.

The three-dimensional distribution chart 310 includes a check box 310a for causing reference areas to be displayed.

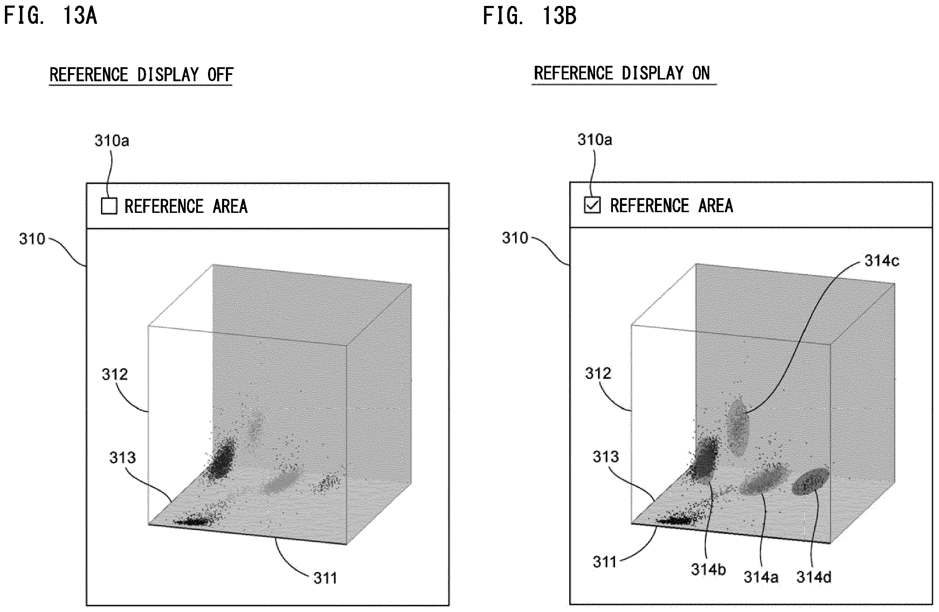

FIGS. 13A and 13B show the three-dimensional distribution charts 310 in the states where display of reference areas is OFF and ON, respectively.

When display of reference areas is OFF, only the dots corresponding to the formed components are displayed on the three-dimensional distribution chart 310 as shown in FIG. 13A. Meanwhile, when the user checks the check box 310a, reference areas indicating distribution of dot clusters are displayed so as to overlap the distribution of the dots in the three-dimensional distribution chart 310, as shown in FIG. 13B. In the example shown in FIG. 13B, the reference areas 314a to 314d are reference areas of neutrophil (NEUT), lymphocyte (LYMPH), monocyte (MONO), and eosinophil (EO), respectively.

Each of the reference areas 314a to 314d is given a color slightly lighter than the color of the corresponding dot cluster. Thus, even when the reference areas 314a to 314d are displayed, the user can grasp to which dot clusters the reference areas 314a to 314d correspond, while grasping the dots on the three-dimensional distribution chart 310.

The reference areas displayed on the three-dimensional distribution chart 310 are distribution areas of dot clusters of a healthy individual. In other words, the reference areas are typical distribution areas (statistically normal distribution areas) of the formed components, and are areas for objectively judging differences from the healthy individual.

Setting of the reference areas may be changed by the user. For example, the reference areas may be set to distribution areas of dot clusters based on the specimen, which was collected a predetermined number of days before or a predetermined number of measurements before, of the subject from whom the specimen displayed on the screen 300 was collected. In this case, the reference areas are areas for objectively judging time-series changes in the formed components contained in the specimen of the subject.

Referring back to FIG. 12, each of the two-dimensional distribution charts 321 to 323 is a two-dimensional distribution chart having, as axes, two parameters out of parameters of the three axes of the three-dimensional distribution chart 310. The two-dimensional distribution charts 321 to 323 are different from each other in combination of the parameters of the two axes. The two-dimensional distribution charts 321 to 323 are disposed in a sub area 305 located at the right end of the screen 300.

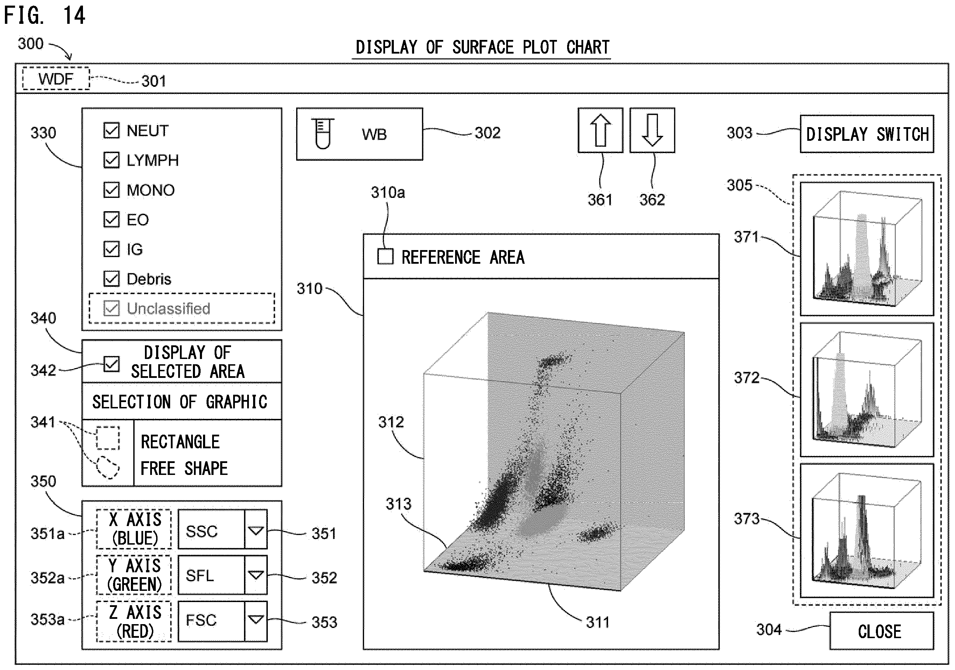

The display switch button 303 is a button for switching the two-dimensional distribution charts 321 to 323 to surface plot charts 371 to 373 (see FIG. 14), respectively. When the user clicks the display switch button 303, the surface plot charts 371 to 373 are displayed in the sub area 305 instead of the two-dimensional distribution charts 321 to 323, respectively.

FIG. 14 schematically shows the configuration of the screen 300 in the state where the surface plot charts 371 to 373 are displayed through an operation performed on the display switch button 303.

In the surface plot charts 371 to 373, a bottom plane (colored plane) is a plane having, as axes, two parameters out of the parameters of the three axes of the three-dimensional distribution chart 310, and the height direction indicates frequency (number). In the two-dimensional distribution charts 321 to 323, since the dots corresponding to the formed components of the same measurement values are rendered at the same coordinate, it is difficult to grasp the dot density. Meanwhile, in the surface plot charts 371 to 373, since the frequency (number) allows the user to grasp how many dots are plotted at the same coordinate, the user can easily grasp the dot density.

Referring back to FIG. 12, in the type selection area 330, a list of the types of formed components classified by the measurement channel of the specimen displayed on the screen 300 is displayed, and each type name is provided with a check box. In the case of the WDF channel, as shown in FIG. 12, neutrophil (NEUT), lymphocyte (LYMPH), monocyte (MONO), eosinophil (EO), immature granulocyte (IG), Debris, and Unclassified are displayed in the type selection area 330. At this time, as for a type of dots in the three-dimensional distribution chart 310 which are unclassified, the type name and the check box are displayed in gray so as not to be checked, as shown by a broken line frame in the type selection area 330. In the initial setting, the check boxes of all the dot types are in the checked state.

When the user unchecks a check box in the type selection area 330, dots of the corresponding type are hidden in the three-dimensional distribution chart 310, the two-dimensional distribution charts 321 to 323, and the surface plot charts 371 to 373. Meanwhile, when the user checks a check box in the type selection area 330, dots of the corresponding type are displayed in the three-dimensional distribution chart 310, the two-dimensional distribution charts 321 to 323, and the surface plot charts 371 to 373.

Display of the dots in the two-dimensional distribution charts 321 to 323 and the surface plot charts 371 to 373 need not be linked with the operation performed on the check box in the type selection area 330.

In the region selection area 340, two icons 341 which allow selection of a rectangle and a free shape, respectively, and a check box 342 which allows selection of display/hiding of a selected region, are displayed. When the user performs an operation of selecting one of the two icons 341, a frame shape, on the screen, for selecting dots in the three-dimensional distribution chart 310 is determined according to the selected icon 341.

After determining the frame shape in the region selection area 340, the user encloses dots to be hidden, in a cube having the three axes of the three-dimensional distribution chart 310. Thus, of the dots in the cube, all the dots present in the enclosed region are selected. Then, the user unchecks the check box 342. Then, the dots selected by the frame shape in the three-dimensional distribution chart 310 are set to be hidden in the three-dimensional distribution chart 310, the two-dimensional distribution charts 321 to 323, and the surface plot charts 371 to 373. When the user checks the check box 342, the dots in the hidden state are displayed again.

Display of the dots in the two-dimensional distribution charts 321 to 323 and the surface plot charts 371 to 373 need not be linked with the operation on the region selection area 340.

The axis selection area 350 includes pull-down menus 351 to 353 which allow selection of parameters of the three axes of the three-dimensional distribution chart 310, and labels 351a to 353a disposed in association with the respective pull-down menus 351 to 353. The pull-down menus 351 to 353 are configured to allow selection of parameters according to the measurement channel. When the screen 300 is newly displayed, default parameters of the three axes of the three-dimensional distribution chart 310 are automatically set, and default values of the pull-down menus 351 to 353 are also automatically set.

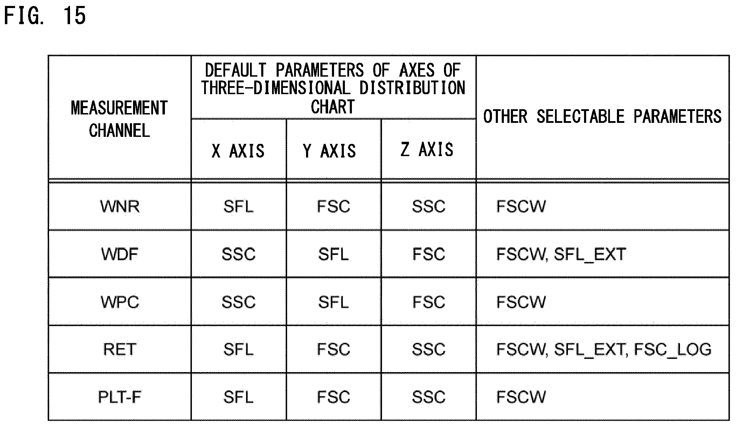

FIG. 15 shows parameters of axes settable on the three-dimensional distribution chart 310 according to the measurement channels, and default parameters set on the three axes of the three-dimensional distribution chart 310.

In the case of the WNR channel, settable parameters are SFL, FSC, SSC, and FSCW, and default parameters set on the X axis, the Y axis, and the Z axis are SFL, FSC, and SSC, respectively. In the case of the WDF channel, settable parameters are SSC, SFL, FSC, FSCW, and SFL_EXT, and default parameters set on the X axis, the Y axis, and the Z axis are SSC, SFL, and FSC, respectively. Regarding the WDF channel, SFL_EXT is settable as an axis only in the body fluid mode (BF). In the case of the WPC channel, settable parameters are SSC, SFL, FSC, and FSCW, and default parameters set on the X axis, the Y axis, and the Z axis are SSC, SFL, and FSC, respectively.

In the case of the RET channel, settable parameters are SFL, FSC, SSC, FSCW, SFL_EXT, and FSC_LOG, and default parameters set on the X axis, the Y axis, and the Z axis are SFL, FSC, and SSC, respectively. In the case of the PLT-F channel, settable parameters are SFL, FSC, SSC, and FSCW, and default parameters set on the X axis, the Y axis, and the Z axis are SFL, FSC, and SSC, respectively.

In the case of FIG. 12, since the screen 300 regarding the WDF channel is newly displayed, the parameters of the respective axes are set according to the initial setting shown in FIG. 15.

Referring back to FIG. 12, when the user changes the parameters of the axes 311 to 313 of the three-dimensional distribution chart 310 by operating the pull-down menus 351 to 353 in the axis selection area 350, two axes of the two-dimensional distribution charts 321 to 323 displayed on the screen 300 or two axes of the surface plot charts 371 to 373 are changed according to the parameters in the pull-down menus 351 to 353.

The axes 311 to 313 of the three-dimensional distribution chart 310 correspond to the X axis, the Y axis, and the Z axis, respectively. The axes 311 to 313 are the parameters displayed in the pull-down menus 351 to 353, respectively. Here, the axes 311 to 313 are blue, green, and red, respectively. The colors of characters in the labels 351a, 352a, and 353a are blue, green, and red, respectively. The character strings in the labels 351a, 352a, and 353a include "blue", "green", and "red", respectively. This allows the user to intuitively grasp which axes of the three-dimensional distribution chart 310 correspond to the axes of the parameters selected in the pull-down menus 351 to 353.

The axes used in the two-dimensional distribution charts 321 to 323 and the surface plot charts 371 to 373 are also color-coded. Between the three-dimensional distribution chart 310, and the two-dimensional distribution charts 321 to 323 and the surface plot charts 371 to 373, the axes of the same parameter are given the same color.

The specimen transition buttons 361 and 362 are buttons for displaying a previous specimen and a next specimen, respectively. When the user clicks the specimen transition button 361 or 362, the specimen displayed on the screen 300 transitions to the previous specimen or the next specimen in the arrangement order of the specimens on the screen 100 shown in FIG. 9, and information on the selected specimen is displayed on the screen 300. When the specimen transition button 361 or 362 on the screen 300 is operated after the arrangement order of the specimens has been changed in advance through setting of filter conditions on the screen 100 shown in FIG. 9, the specimen to be displayed on the screen 300 transitions to the previous specimen or the next specimen on the basis of the arrangement order having been changed on the screen 100.

The close button 304 is a button for closing the screen 300. When the user clicks the close button 304, the screen 300 is closed, and the screen on which the operation of opening the screen 300 was performed (e.g., the screen 200 shown in FIG. 10 or FIG. 11) is displayed on the display 23.

When the close button 304 is operated, all the display conditions (the selection state in the type selection area 330, the selection and display states in the region selection area 340, the selection state of the three parameters in the axis selection area 350, the view angle of the three-dimensional distribution chart 310, and the display states of the two-dimensional distribution charts 321 to 323 or the surface plot charts 371 to 373) on the screen 300 are reset. When the screen 300 is displayed next, this screen 300 is displayed with default display conditions. Specifically, all the check boxes in the type selection area 330 are checked, the region selected in the region selection area 340 is canceled, the parameters of the three axes in the axis selection area 350 are set to the default values shown in FIG. 15, the view angle of the three-dimensional distribution chart 310 is set at a default angle, and the two-dimensional distribution charts 321 to 323 are displayed in the sub area 305.

Unless the close button 304 is operated, the display state of the screen 300 is maintained for the same measurement channel and the same measurement mode even if the specimen transition button 361 or 362 is operated. The display state being maintained will be described later with reference to Display Example 2.

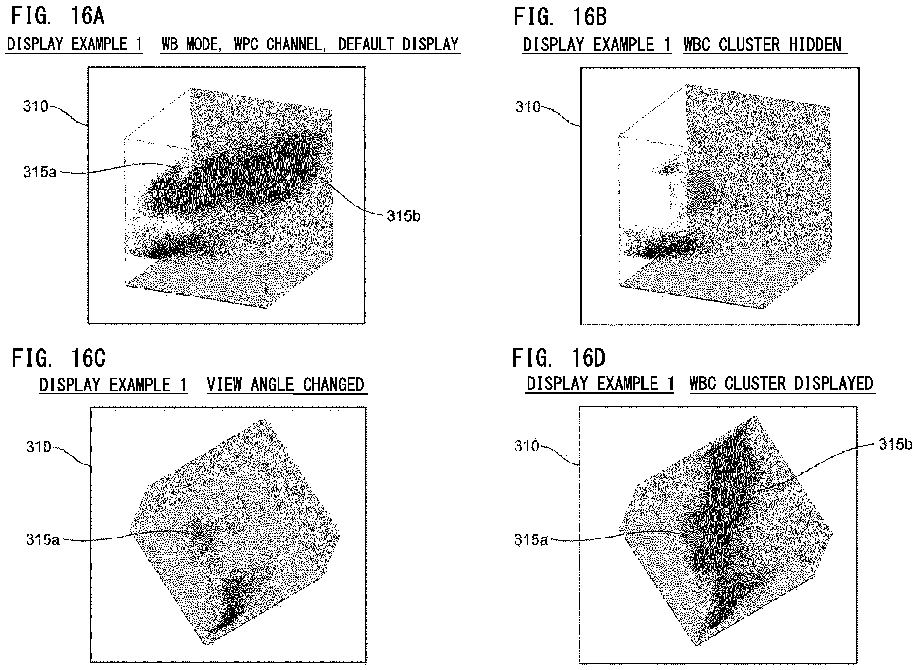

Next, Display Examples 1 to 5 of the screen 300 and the three-dimensional distribution chart 310 will be described with reference to FIG. 16A to FIG. 26D.

FIGS. 16A to 16D each show a three-dimensional distribution chart 310 according to Display Example 1. In Display Example 1, the measurement mode is the whole blood mode (WB), and the measurement channel is the WPC channel.

As shown in FIG. 16A, when the view angle is a default, a dot group 315a of immature cells and abnormal cells is hidden behind a dot group 315b of white blood cells widely spreading frontward, and therefore, it is difficult for the user to confirm distribution of the immature cells and the abnormal cell. When a plurality of different types of dots are present at the same coordinate, since only a type of dots larger in number at the coordinate is displayed, the user cannot confirm a type of dots smaller in number. In the example of FIG. 16A, since the dot group 315b is larger in number, the dot group 315a located at the same position is not likely to be displayed.

Meanwhile, the user can hide the dot group 315b of white blood cells by operating the check box in the type selection area 330 shown in FIG. 12. Thus, as shown in FIG. 16B, the user can confirm the dot group 315a of immature cells and abnormal cells having been hidden behind the dot group 315b of white blood cells. Moreover, the user can confirm the dot group 315b of immature cells and abnormal cell that has been present at the same coordinate as white blood cells and therefore has not been displayed.

The user can change the view angle of the three-dimensional distribution chart 310 from the state of FIG. 16B to make distribution of the dot group 315a of immature cells and abnormal cells more easily viewable as shown in FIG. 16C.

Moreover, the user operates the check box in the type selection area 330 shown in FIG. 12 from the state of FIG. 16C to cause the dot group 315b of white blood cells to be displayed again. Thus, the user can grasp the positional relationship between the dot group 315a of immature cells and abnormal cells and the dot group 315b of white blood cells as shown in FIG. 16D.

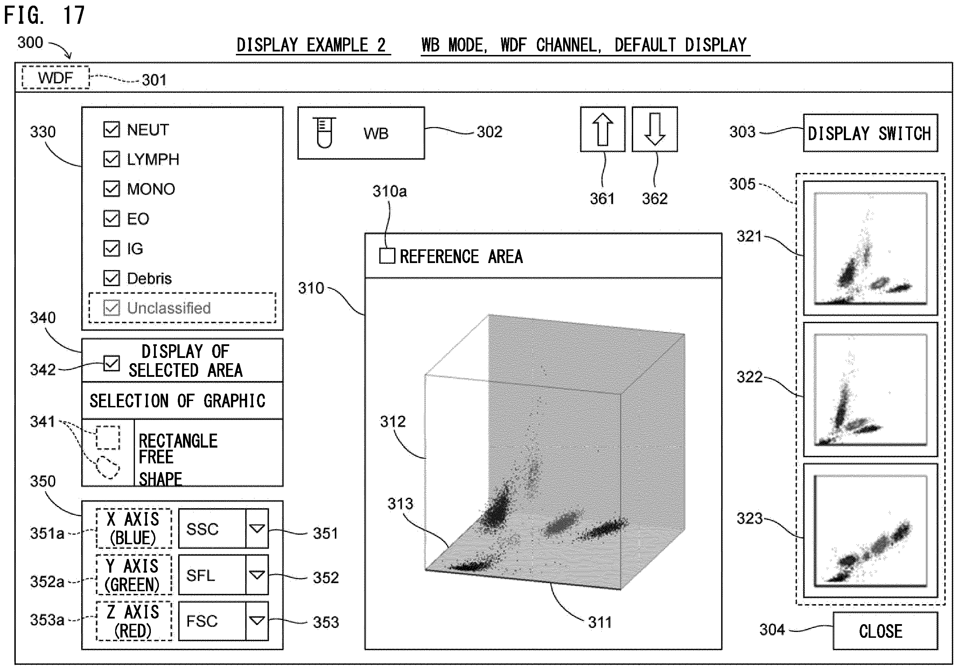

FIGS. 17 to 23 each schematically show a screen 300 according to Display Example 2.

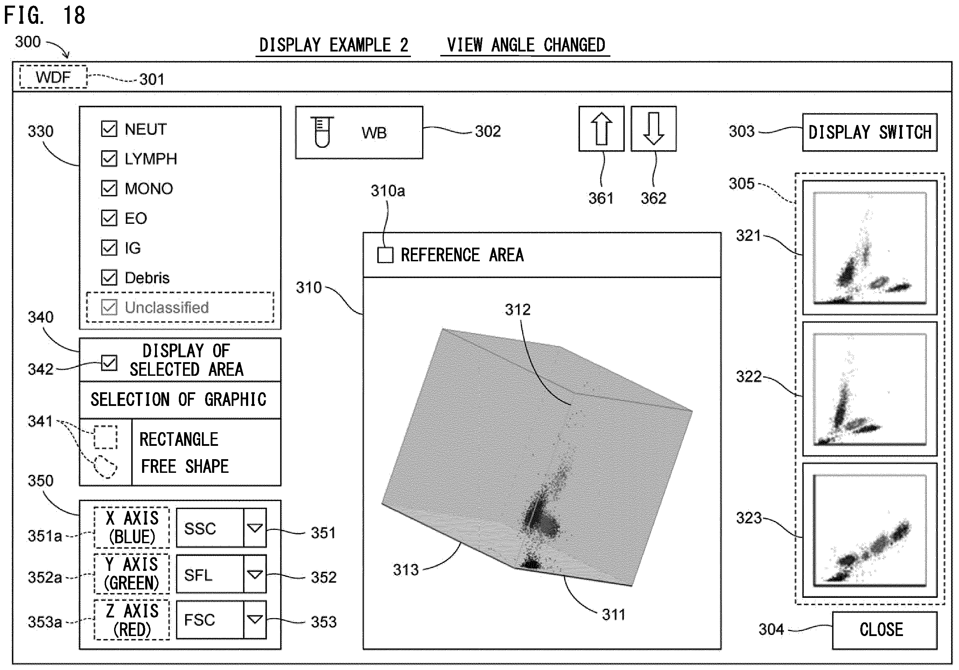

When the user double-clicks the two-dimensional distribution chart 231 in FIG. 10, the screen 300 is displayed with default display conditions. In FIG. 17, the measurement mode is the whole blood mode (WB), and the measurement channel is the WDF channel. In this state, the user performs a drag operation on the display area in the three-dimensional distribution chart 310 to change the view angle as shown in FIG. 18.

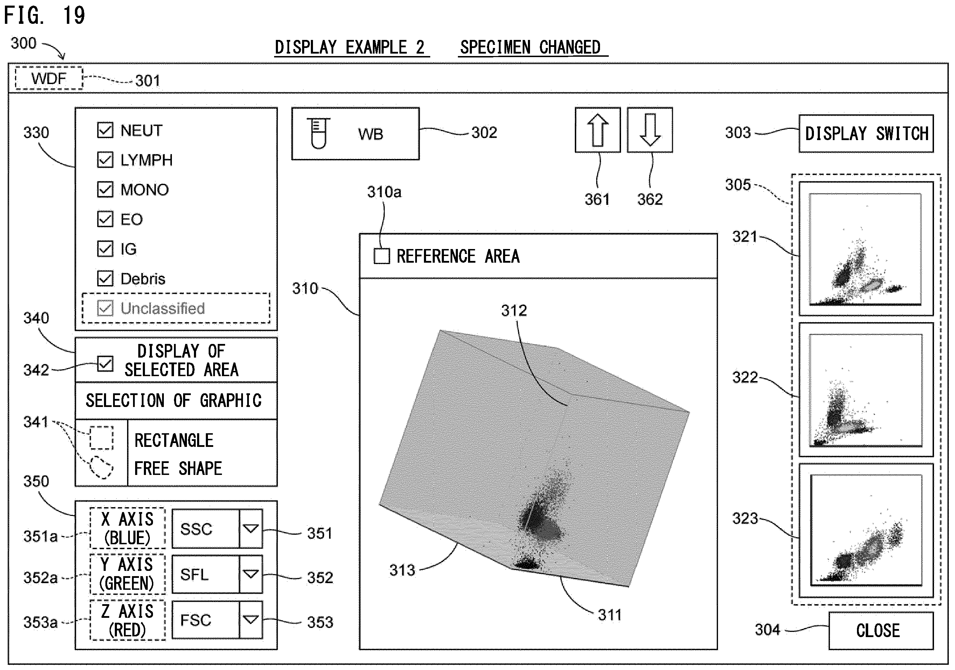

Subsequently, on the screen 300 shown in FIG. 18, when the user clicks the specimen transition button 361 or 362, the specimen to be displayed on the screen 300 is changed according to the arrangement order of specimens in the measurement data display area 110 shown in FIG. 9, as shown in FIG. 19. At this time, if the measurement mode and the measurement channel of the screen 300 before transition shown in FIG. 18 are the same as the measurement mode and the measurement channel of the screen 300 after transition shown in FIG. 19, the display conditions of the screen 300 are maintained.

More specifically, in the case where the measurement mode of the specimen before transition is the same as the measurement mode after transition and there is, regarding the specimen after transition, measurement data of the same measurement channel as the measurement channel displayed on the screen 300 before transition shown in FIG. 18, the display conditions of the screen 300 after transition shown in FIG. 19 are set to be the same as the display conditions of the screen 300 before transition shown in FIG. 18. Thus, as for the specimens having the same measurement conditions (having the same measurement mode and the same measurement channel), the user can confirm the three-dimensional distribution chart 310 with the same display conditions.

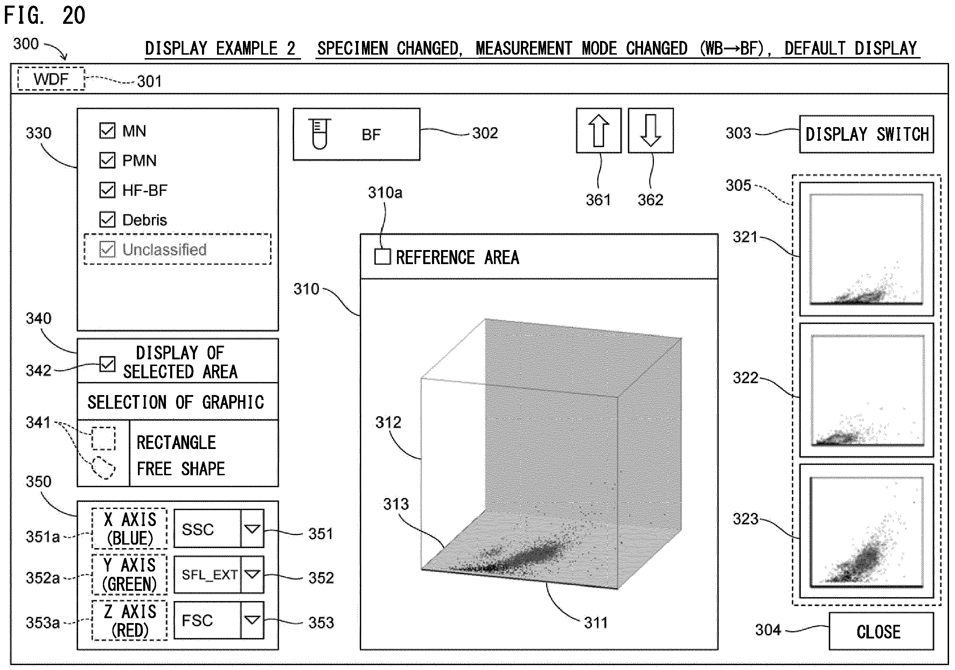

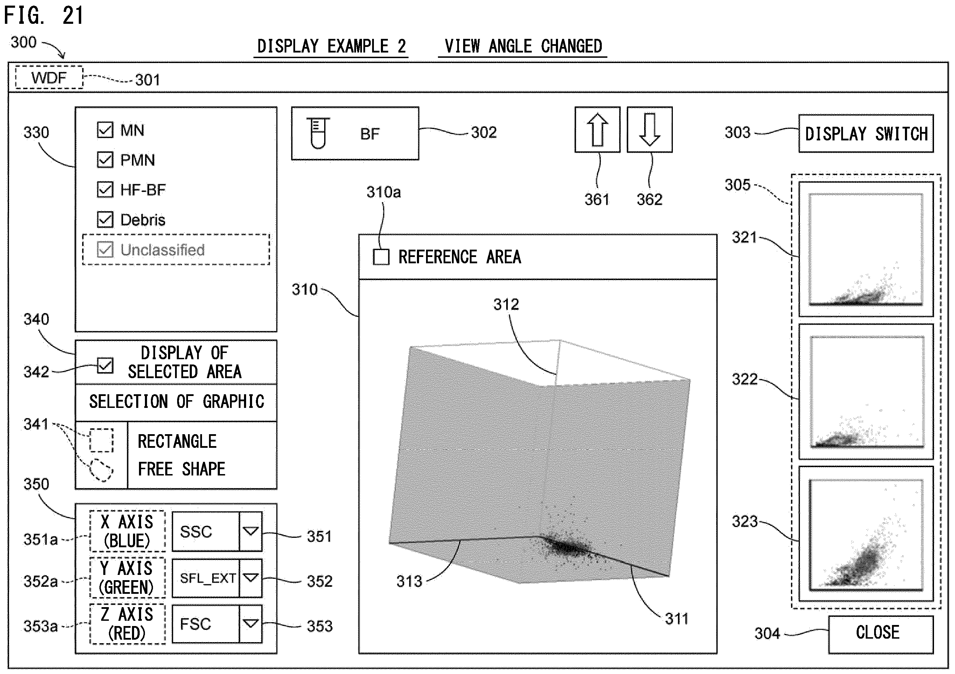

Subsequently, on the screen 300 shown in FIG. 19, when the user clicks the specimen transition button 361 or 362, the specimen to be displayed on the screen 300 is changed as shown in FIG. 20. In FIG. 20, the measurement mode is the body fluid mode (BF), and the measurement channel is the WDF channel. Since the measurement mode is different from that of the screen 300 before transition shown in FIG. 19, the screen 300 is displayed with the display conditions for the body fluid mode (BF) and the WDF channel (in FIG. 20, default display conditions). From this state, the user performs a drag operation on the display area in the three-dimensional distribution chart 310, whereby the user can change the view angle as shown in FIG. 21.

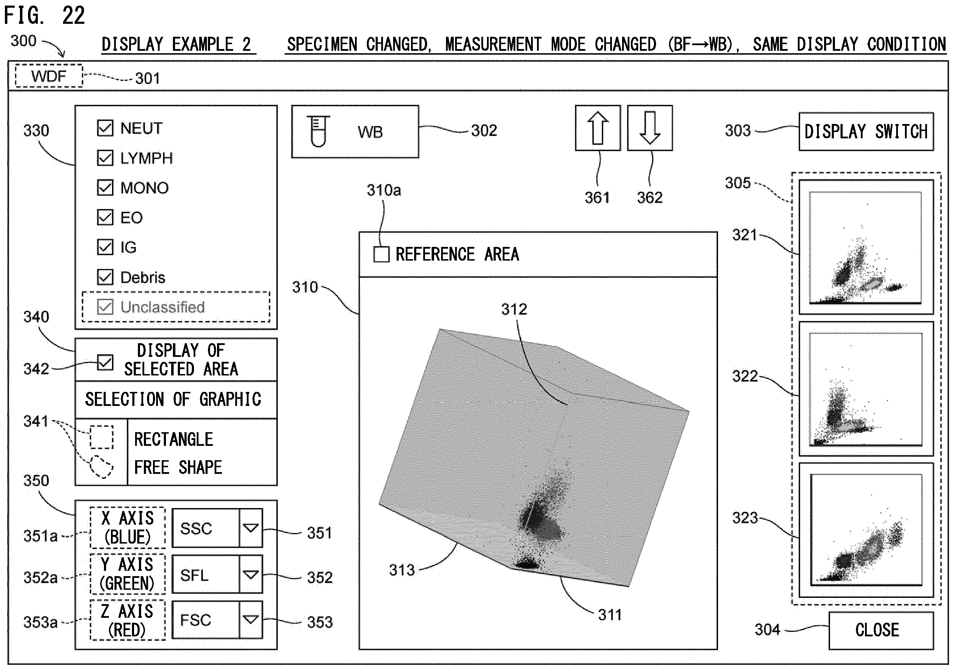

Subsequently, on the screen 300 shown in FIG. 21, when the user clicks the specimen transition button 361 or 362 to cause the specimen shown in FIG. 19 to be displayed again on the screen 300, the screen 300 is displayed, as shown in FIG. 22, with the same display conditions as those shown in FIG. 19. That is, the display conditions for the whole blood mode (WB) and the WDF channel are maintained as the display conditions of the screen 300 shown in FIG. 19. Therefore, when display is performed again with the same measurement mode and the same measurement channel as shown in FIG. 22, the screen 300 is displayed with the same display conditions.

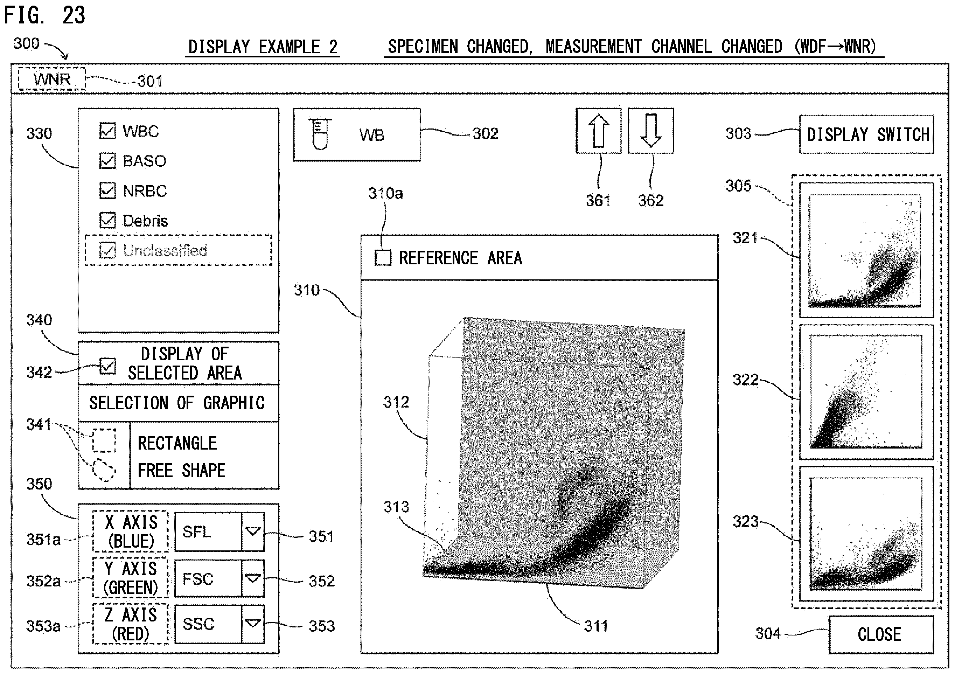

Subsequently, on the screen 300 shown in FIG. 22, when the user clicks the specimen transition button 361 or 362, the specimen to be displayed on the screen 300 is changed as shown in FIG. 23. In FIG. 23, the measurement mode is the whole blood mode (WB), and the measurement channel is the WNR channel. Since the measurement channel is different from that of the screen 300 before transition shown in FIG. 22, the screen 300 is displayed with the display conditions for the whole blood mode (WB) and the WNR channel (in FIG. 23, default display conditions).

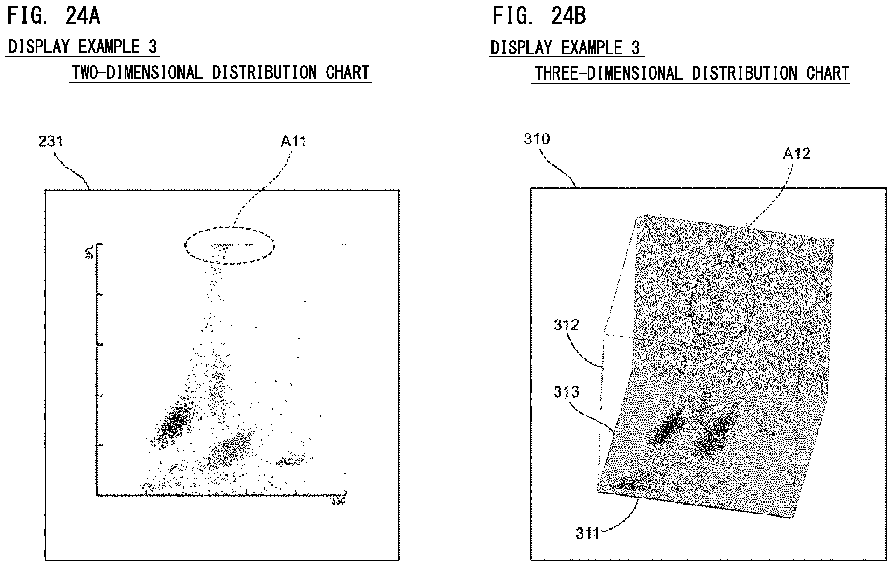

FIGS. 24A and 24B show a two-dimensional distribution chart 231 and a three-dimensional distribution chart 310 according to Display Example 3.

FIG. 24A shows the two-dimensional distribution chart 231 regarding the WDF channel displayed on the screen 200 shown in FIG. 10. In this case, in an upper-limit area A11 of the vertical axis (SFL) of the two-dimensional distribution chart 231, dots corresponding to antibody-producing cells having a large amount of protein may be distributed. Thus, when the dots are concentrated at an end in the axial direction, the user cannot easily grasp how many dots are distributed in this area.

Meanwhile, the user double-clicks the two-dimensional distribution chart 231 to cause the screen 300 to be displayed, and adjusts the view angle of the three-dimensional distribution chart 310 as shown in FIG. 24B. This allows the user to grasp in more detail the dots concentrated at the end in the axial direction. That is, the user confirms distribution of the dots in the area A12 corresponding to the area A11, with reference to the three-dimensional distribution chart 310 shown in FIG. 24B. Thus, the user can grasp how the dots are distributed toward the upper limit of the vertical axis (SFL), and can grasp how many dots are distributed in the area A12.

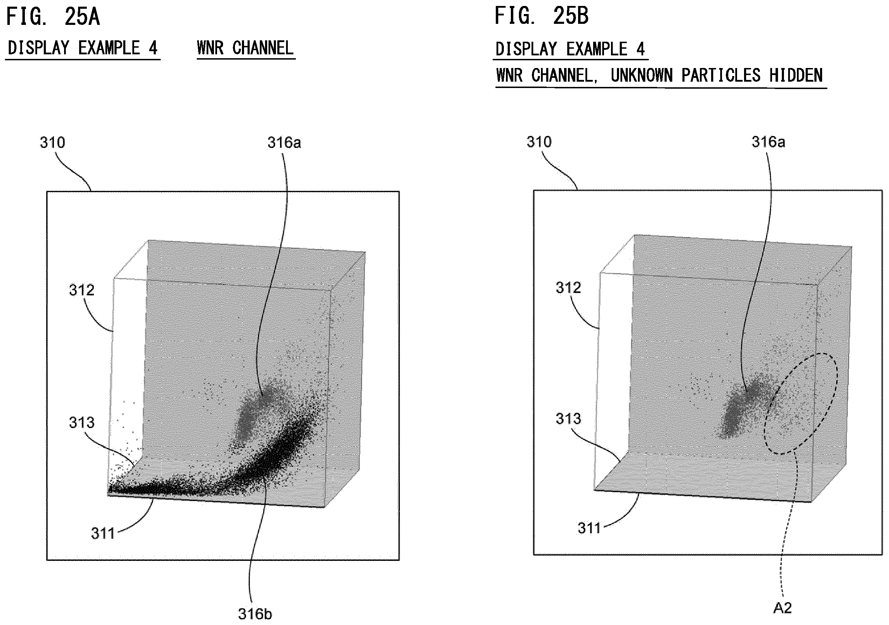

FIGS. 25A and 25B each show a three-dimensional distribution chart 310 according to Display Example 4.

FIG. 25A shows the three-dimensional distribution chart 310 regarding the WNR channel. In the example shown in FIG. 25A, a dot group 316a of white blood cells is hidden behind a dot group 316b of unknown particles widely spreading frontward. In addition, when dots of white blood cells and dots of unknown particles are present at the same coordinate, the user cannot confirm the dots of white blood cells even when the view angle of the three-dimensional distribution chart 310 is changed.

Meanwhile, the user can hide the cluster (dot group) of unknown particles as shown in FIG. 25B by operating the check box (see FIG. 12) in the type selection area 330 in the screen 300. This allows the user to confirm in detail the dot group 316a of white blood cells near the area A2 which had been overlapped with the dots of unknown particles.

When the dot groups 316a and 316b are distributed as shown in FIG. 25A, the user can confirm the dot group 316a of white blood cells near the area A2 through another procedure. That is, the user hides the white blood cells by operating the check box in the type selection area 330, and encloses the area A2 and its vicinity with a rectangle or a free shape selected by operating the icon 341 (see FIG. 12) in the region selection area 340 in the screen 300. Subsequently, the user unchecks the check box 342 in the region selection area 340 in the screen 300 to hide the dots of unknown particles present near the area A2. Then, the user causes the dot group 316a of white blood cells to be displayed again by checking the check box in the type selection area 330. This allows the user to confirm in detail the dot group 316a of white blood cells near the area A2 which had been overlapped with the dot group 316b of unknown particles.

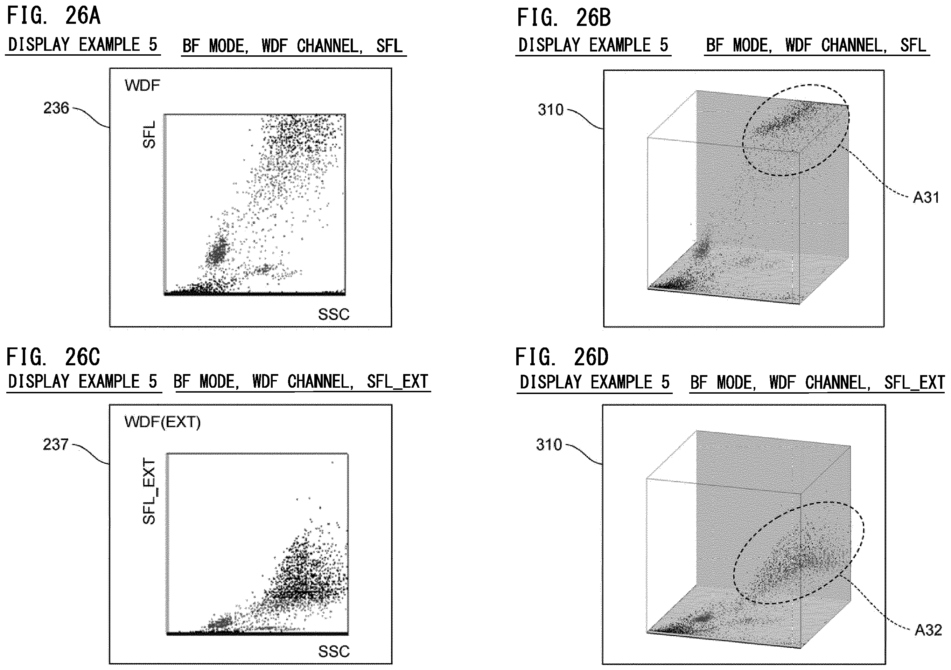

FIGS. 26A to 26D show two-dimensional distribution charts 236 and 237 and three-dimensional distribution charts 310 according to Display Example 5.

As described with reference to FIG. 11, as for the WDF channel in the body fluid mode (BF), there are SFL at a normal scale and SFL_EXT at a widely extended scale, as parameters regarding SFL.

FIG. 26A shows the two-dimensional distribution chart 236 on the screen 200 shown in FIG. 11. In the two-dimensional distribution chart 236, the vertical axis is the normal-scale SFL. When the user double-clicks the two-dimensional distribution chart 236, the three-dimensional distribution chart 310 shown in FIG. 26B is displayed. At this time, the vertical axis of the three-dimensional distribution chart 310 is the SFL at the same scale as that of the two-dimensional distribution chart 236.

FIG. 26C shows the two-dimensional distribution chart 237 on the screen 200 shown in FIG. 11. In the two-dimensional distribution chart 237, the vertical axis is the SFL_EXT at the widely extended scale. When the user double-clicks the two-dimensional distribution chart 237, the three-dimensional distribution chart 310 shown in FIG. 26D is displayed. At this time, the vertical axis of the three-dimensional distribution chart 310 is the SFL_EXT at the same scale as that of the two-dimensional distribution chart 237.

In the case of the three-dimensional distribution chart 310 shown in FIG. 26B, the user cannot appropriately grasp the shape of a dot group affixed to the upper limit of the vertical axis (SFL), that is, how the dot group extends in an area A31 and its vicinity. However, in the three-dimensional distribution chart 310 shown in FIG. 26D, an area A32 corresponds to the area A31. Therefore, the three-dimensional distribution chart 310 shown in FIG. 26D allows the user to appropriately grasp the shape of the dot group in the area A32 and its vicinity.

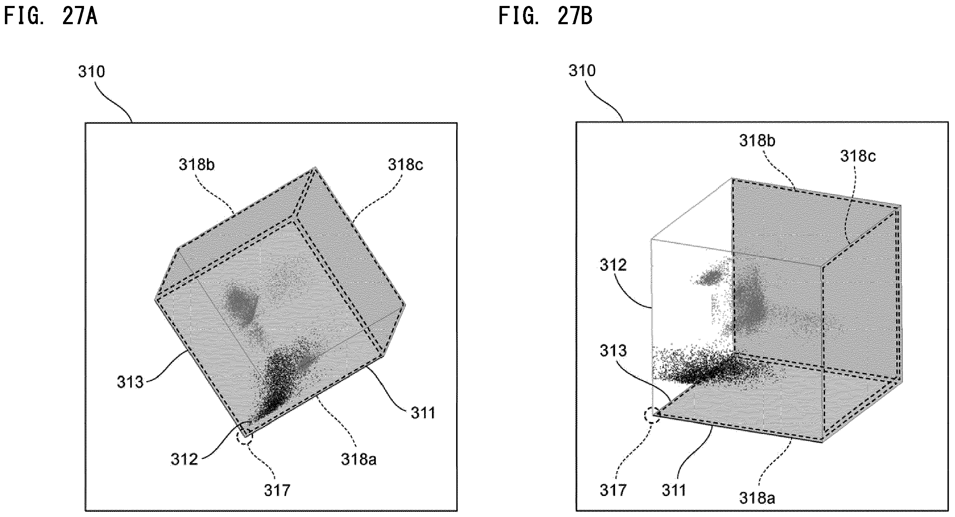

Next, the colors of the planes of the rectangular parallelepiped shape of the three-dimensional distribution chart 310 will be described with reference to FIGS. 27A and 27B. FIGS. 27A and 27B are similar to FIGS. 16C and 16B, respectively.

As shown in FIG. 27A, the three-dimensional distribution chart 310 is provided with axes 311 to 313 corresponding to the X axis, the Y axis, and the Z axis, respectively. A point at which the axes 311 to 313 intersect each other is an origin 317. Of the six planes of the rectangular parallelepiped shape, the inner sides of three planes 318a, 318b, and 318c are given different colors. The plane 318a is a plane formed by the axis 311 and the axis 313. The plane 318b is a plane that is parallel to a plane formed by the axis 311 and the axis 312 and does not pass the origin 317. The plane 318c is a plane that is parallel to a plane formed by the axis 312 and the axis 313 and does not pass the origin 317.

Thus, since the planes 318a, 318b, and 318c, of the six planes of the three-dimensional distribution chart 310, are colored, the user can easily grasp the view angle at which the three-dimensional distribution chart 310 is displayed. Moreover, since only the three planes out of the six planes are colored, display of the three-dimensional distribution chart 310 is avoided from being complicated.

Depending on the view angle of the three-dimensional distribution chart 310, not the inner face but the outer face of the plane 318a, 318b, or 318c may be displayed. For example, in the case of the view angle shown in FIG. 27B, the outer face of the plane 318c is displayed. Thus, a plane, among the planes 318a, 318b, and 318c, whose outer face is displayed is made transparent (transparently displayed). In this case, the colors of the planes 318a, 318b, and 318c are not overlapped, thereby avoiding display of the three-dimensional distribution chart 310 from being complicated.

### <Effect of Embodiment 1>

According to Embodiment 1, the following effects are achieved.

In step S14, the measurement values of at least three parameters are obtained from each formed component. In step S1741, the screen 300 including the three-dimensional distribution chart 310 in which the three parameters are set as the axes 311 to 313 and the formed components are represented by dots, is displayed. In step S1743, specific dots, of the dots on the three-dimensional distribution chart 310, are hidden, in response to an operation performed by the user.

According to the above process, the user can hide the dots, which are not required for analysis of a desired formed component, on the three-dimensional distribution chart 310. Thus, the dots of the formed component desired by the user can be displayed on the three-dimensional distribution chart 310 in an easily viewable manner, thereby improving visibility of distribution of the formed component on the three-dimensional distribution chart 310. Moreover, in validation of the data (measurement values) corresponding to the measurement items displayed in the measurement item display area 120, the user, by using the three-dimensional distribution chart 310, can accurately grasp: presence/absence of immature cells or abnormal cells; and the positional relationship and distribution of the dot groups corresponding to the respective formed components. This realizes more smooth and accurate analysis of the formed components.

In step S16, on the basis of the measurement values, the formed components are classified into a plurality of types as indicated by the classification values shown in FIG. 5. In step S1742, selection of any of the classified formed component types is received through the type selection area 330. In step S1743, specific dots are hidden for each of the formed component types having been selected. Thus, the dots on the three-dimensional distribution chart 310 can be hidden for each of the formed component types. For example, when dot groups of different types overlap each other on the three-dimensional distribution chart 310, the dot group of one of the types can be hidden, whereby the dot group of the other type can be easily grasped. This allows the user to smoothly and accurately advance analysis on the desired formed component type.

In step S174, while the specific dots are hidden, the view angle of the three-dimensional distribution chart 310 is changed according to an operation performed on the three-dimensional distribution chart 310. Thus, the user can grasp in detail distribution of desired dots by changing the view angle of the three-dimensional distribution chart 310.

In step S174, the dots on the three-dimensional distribution chart 310 are color-coded for each type. This allows the user to visually and easily grasp the dots corresponding to the respective types.

In the case where dots are hidden, selection of dots to be displayed on the three-dimensional distribution chart 310 is received through the type selection area 330 or the region selection area 340. Thus, the dots, which are in the hidden state, can be displayed again.

In step S1742, selection of specific dots to be hidden on the three-dimensional distribution chart 310 is received through the type selection area 330 or the region selection area 340. This allows the user to easily view desired dots, and smoothly and accurately advance analysis of the dots.

In step S173, the plurality of two-dimensional distribution charts 231 to 237 corresponding to different measurement channels are displayed. Any of the two-dimensional distribution charts 231 to 237 shown in FIGS. 10 and 11 is selected in response to an operation performed on the two-dimensional distribution chart. The three-dimensional distribution chart 310 displayed in step S174 is the three-dimensional distribution chart 310 corresponding to the measurement channel of the selected two-dimensional distribution chart (any of 231 to 237). This allows the user to smoothly refer to the three-dimensional distribution chart 310 of the predetermined measurement channel while referring to the two-dimensional distribution chart (any of 231 to 237) of the measurement channel.

In step S174, the three-dimensional distribution chart 310 is displayed by using the axis at the same scale as the axis used in the selected two-dimensional distribution chart (any of 231 to 237). For example, as shown in FIGS. 26A and 26B, the vertical axis of the three-dimensional distribution chart 310 and the vertical axis of the two-dimensional distribution chart 236 are axes at the same scale (SFL at the normal scale). As shown in FIGS. 26C and 26D, the vertical axis of the three-dimensional distribution chart 310 and the vertical axis of the two-dimensional distribution chart 237 are axes at the same scale (SFL_EXT at the widely extended scale). This allows the user to perform analysis of the formed component by using the three-dimensional distribution chart 310 similarly to the two-dimensional distribution charts 231 to 237.

In step S174, three axes of the three-dimensional distribution chart 310 are selected from among a plurality of (four or more) parameters through the axis selection area 350. Thus, the user can cause the three-dimensional distribution chart 310 having, as the three axes, any parameters selected from the plurality of parameters to be displayed. This allows the user to perform, in more detail, analysis of the formed component by using the three-dimensional distribution chart 310.

The three axes used in the three-dimensional distribution chart 310 are color-coded, and the colors of the respective axes and the names of the parameters set on the axes are displayed in the axis selection area 350. This allows the user to smoothly grasp which axis of the three-dimensional distribution chart 310 corresponds to which parameter, even when the view angle of the three-dimensional distribution chart 310 is changed.

In step S174, the display conditions (display state of dots, parameters of axes, and view angle) of the three-dimensional distribution chart 310 are set for each specimen measurement mode. For example, when the specimen transition button 361 or 362 is operated on the screen 300 shown in FIG. 19 and thereby another specimen is displayed on the screen 300 as shown in FIG. 20, since the measurement mode shown in FIG. 19 is different from the measurement mode shown in FIG. 20, the display conditions are initialized on the screen 300 shown in FIG. 20. Thus, the user can cause the three-dimensional distribution chart 310 to be displayed with preset optimum display conditions for each specimen measurement mode. This allows the user to smoothly start analysis of the formed component using the three-dimensional distribution chart 310.

In step S174, the display conditions (display state of dots, parameters on axes, and view angle) of the three-dimensional distribution chart 310 are set for each measurement channel to be used in step S14 of obtaining the measurement values. For example, when the specimen transition button 361 or 362 is operated on the screen 300 shown in FIG. 22 and thereby another specimen is displayed on the screen 300 as shown in FIG. 23, since the measurement channels shown in FIG. 22 and FIG. 23 are different from each other, the display conditions are initialized on the screen 300 shown in FIG. 23. Thus, the user can cause the three-dimensional distribution chart 310 to be displayed with preset optimum display conditions for each measurement channel. This allows the user to smoothly start analysis of the formed component using the three-dimensional distribution chart 310.

In step S174, the two-dimensional distribution charts 321 to 323 or the surface plot charts 371 to 373 corresponding to the three-dimensional distribution chart 310 are displayed in an area (sub area 305) different from the area, in the screen 300, where the three-dimensional distribution chart 310 is displayed. This allows the user to analyze in more detail the formed component while referring to the two-dimensional distribution charts 321 to 323 or the surface plot charts 371 to 373 as well as the three-dimensional distribution chart 310.

In step S174, two parameters, out of the three parameters used as the three axes of the three-dimensional distribution chart 310, are set as the parameters of each of the two-dimensional distribution charts 321 to 323 or the surface plot charts 371 to 373. In this case, on one screen 300, the two-dimensional distribution charts 321 to 323 or the surface plot charts 371 to 373 are related to the three-dimensional distribution chart 310, which allows the user to smoothly analyze the formed component by using the two-dimensional distribution charts 321 to 323 or the surface plot charts 371 to 373.

In step S174, setting of the parameters to be used as the axes of the three-dimensional distribution chart 310 is received through the axis selection area 350, and the parameters of the two-dimensional distribution charts 321 to 323 or the surface plot charts 371 to 373 are changed on the basis of the received parameters. In this case, when the user has changed the parameters of the axes of the three-dimensional distribution chart 310, the parameters of the two-dimensional distribution charts 321 to 323 or the surface plot charts 371 to 373 are changed in accordance with the parameters of the three-dimensional distribution chart 310. Thus, the user can compare the three-dimensional distribution chart 310 with the two-dimensional distribution charts 321 to 323 or the surface plot charts 371 to 373 by using the same parameters, and therefore can smoothly compare the respective figures.

In step S174, in response to the step of receiving designation of hiding specific dots, the dots, on the two-dimensional distribution charts 321 to 323 or the surface plot charts 371 to 373, corresponding to the specific dots to be hidden are also hidden. In this case, since the same dots are deleted from the three-dimensional distribution chart 310 and the two-dimensional distribution charts 321 to 323 or the surface plot charts 371 to 373, the user can smoothly analyze the formed component by using the three-dimensional distribution chart 310 and the two-dimensional distribution charts 321 to 323 or the surface plot charts 371 to 373.

The axes used in the three-dimensional distribution chart 310 and the two-dimensional distribution charts 321 to 323 or the surface plot charts 371 to 373 are color-coded such that the axes of the same parameter are given the same color between the three-dimensional distribution chart 310 and the two-dimensional distribution charts 321 to 323 or the surface plot charts 371 to 373. In this case, in the three-dimensional distribution chart 310 and the two-dimensional distribution charts 321 to 323 or the surface plot charts 371 to 373, the user can grasp the axes in a distinguishable manner, and can smoothly perform analysis based on the respective parameters.

In the three-dimensional distribution chart 310, the reference areas corresponding to the clusters of the formed components are displayed so as to overlap the clusters. In the example shown in FIG. 13B, the reference areas 314a to 314d are displayed so as to correspond to the clusters of the formed components. This allows the user to analyze in more detail the formed components by comparing the reference areas with distribution of the dots on the three-dimensional distribution chart 310.

Specifically, in the case where the reference areas are areas for objectively judging differences from a healthy individual, the user can relatively grasp the distribution states of the respective formed components of the specimen by comparing the reference areas with distribution of the formed components of the specimen. This allows pathologic diagnosis based on the formed components of the specimen to smoothly advance. Meanwhile, in the case where the reference areas are areas for objectively judging time-series changes in the respective formed components contained in the specimen of the subject, the user can judge change in the disease condition of the subject and determine an administration strategy or the like by grasping how the distribution states of the formed components in the specimen change.

The three-dimensional distribution chart 310 is switched to the three-dimensional distribution chart 310 of another specimen by an operation performed on the specimen transition button 361 or 362. Thus, the user can easily compare the three-dimensional distribution chart 310 of one specimen with the three-dimensional distribution chart 310 of the other specimen, and therefore can analyze in more detail the formed components of one or both of the specimens.

The three-dimensional distribution chart 310 of the other specimen is displayed with the display conditions before switching being maintained. For example, in the case where the specimen transition button 361 or 362 is operated on the screen 300 shown in FIG. 18 and thereby another specimen is displayed on the screen 300 as shown in FIG. 19, since the measurement mode and the measurement channel shown in FIG. 18 are the same as those shown in FIG. 19, the screen 300 of FIG. 19 is displayed with the display conditions of the screen 300 of FIG. 18 being maintained. Thus, the user can cause the three-dimensional distribution chart 310 of one specimen and the three-dimensional distribution chart 310 of the other specimen to be displayed with the same display conditions, and therefore can smoothly compare the three-dimensional distribution charts 310.

As shown in FIG. 16A to FIG. 26D, the user can confirm the distribution shapes of the clusters of the formed components by referring to the three-dimensional distribution chart 310 displayed on the screen 300. The distribution shapes thus confirmed can be effectively used for judging the condition of the subject from whom the specimen was collected.

In step S14, the measurement values are obtained from each formed component. In step S16, the measurement data (data set) including at least three parameters based on the measurement values and the attribute of the formed component (classification value shown in FIG. 5) are obtained. In step S1741, on the basis of the measurement data (data set), the screen 300 including the three-dimensional distribution chart 310 in which the formed component is represented by dots is displayed. In step S1743, the dots on the three-dimensional distribution chart 310 are displayed or hidden on the basis of the attribute of the formed component.

According to the above process, the dots, which are not required for analysis of a predetermined formed component, are displayed or hidden on the three-dimensional distribution chart 310. Thus, the dots of the predetermined formed component can be displayed on the three-dimensional distribution chart 310 in an easily viewable manner, thereby improving visibility of distribution of the formed component on the three-dimensional distribution chart 310. Moreover, in validation of the data (measurement values) corresponding to the measurement items displayed in the measurement item display area 120, the user, by using the three-dimensional distribution chart 310, can accurately grasp: presence/absence of immature cells or abnormal cells; and the positional relationship and distribution of the dot groups corresponding to the respective formed components. This realizes more smooth and accurate analysis of the formed components.

### <Modification 1: modification regarding dot designating method>

In Embodiment 1 described above, in order to designate a dot group to be subjected to display/hiding switching, an example of designating the type of a component and an example of designating a range of dots present at a specific coordinate, have been described. Meanwhile, another method may be adopted for designating a dot group to be subjected to display/hiding switching.

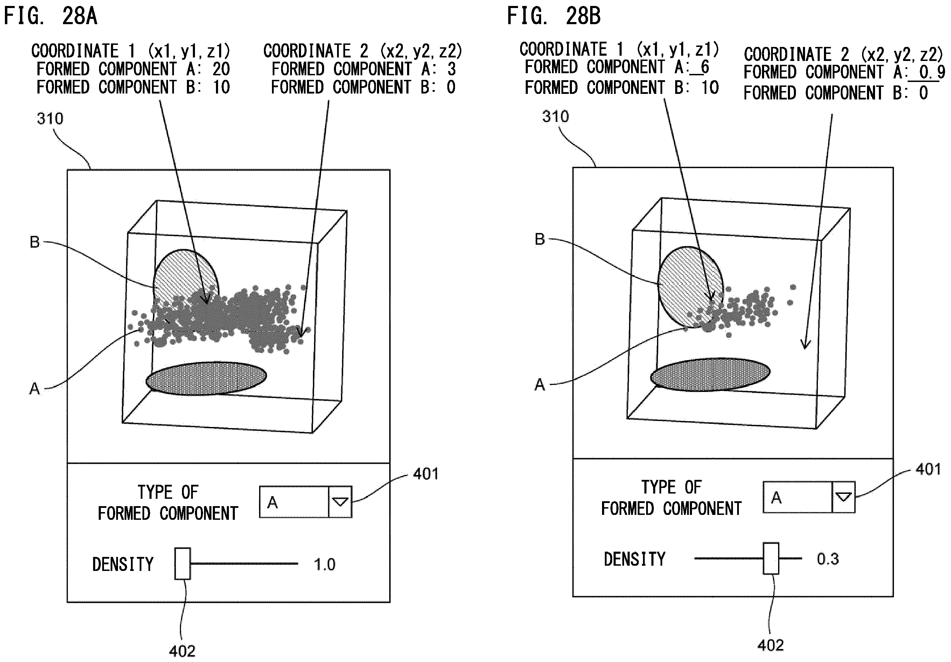

FIGS. 28A and 28B each schematically show a three-dimensional distribution chart 310 according to Modification 1.

In Modification 1, a pull-down menu 401 for receiving selection of a type of a formed component the display density of which is to be changed, and a slider bar 402 (hereinafter referred to as "bar") for changing the display density of dots to be hidden, are displayed together with the three-dimensional distribution chart 310. In Modification 1, the density of dots can be changed by operating the displayed bar, and the number of dots to be displayed in response to the change in the density can be continuously changed within a range of 0 to 1. In FIG. 28A, the type of the target formed component, the dot density of which is to be changed, is set to "A". In addition, the density is set at 1.0, which means that the dots of the target formed component are 100% displayed. When the user changes the density to 0.3 by operating the bar, 70% of the target formed component is hidden while only the remaining 30% is displayed as shown in FIG. 28B. That is, according to the example shown in FIGS. 28A and 28B, the user can switch display/hiding of dots by designating the density of the dots instead of designating a specific formed component or specific dots.

Display of the dots according to the designated density can be implemented by, for example, multiplying the frequency of each dot by a coefficient according to the density. For example, in FIG. 28A, when the density of the formed component classified as the type A (hereinafter referred to as "formed component A") is changed to 0.3, the frequency is recalculated with a value obtained by multiplying the frequency of the formed component A at each coordinate by the coefficient of 0.3. For example, at a coordinate 1, the frequency of the formed component A is reduced from 20 to 6 and becomes lower than 10 which is the frequency of a formed component, at the same coordinate, classified as a type B (hereinafter referred to as "formed component B"). Therefore, the color of the dot at the coordinate 1 is changed from the color of the formed component A to the color of the formed component B. Moreover, at a coordinate 2, the frequency of the formed component A is 0.9 which is less than 1, and therefore, the corresponding dot disappears. By multiplying the frequency of each dot by the coefficient according to the density, the frequencies of all the dots can be uniformly changed.

Thus, the dots of the formed component B, which were not confirmable due to the formed component A as shown in FIG. 28A, become confirmable as shown in FIG. 28B.

The display/hiding switching may be performed not by uniformly changing the dot frequency but by changing a threshold according to the dot frequency. For example, in accordance with the density designated by the bar shown in FIGS. 28A and 28B, a percentile using, as a score, the dot frequency may be designated as a threshold. In this case, when the designated density is 0.3, dots up to the 70th percentile in ascending order of the dot frequency are hidden. The threshold for the display change may be a percentile, an absolute value of a frequency with respect to a coordinate, or a relative value with respect to a maximum frequency.

As described above, according to Modification 1, the density of dots to be displayed can be changed. For example, even when dots of a first formed component are thick and therefore dots of a second formed component are hidden behind the dots of the first formed component, distribution of the second formed component can be made easily viewable by reducing the density of the dots of the first formed component.

### <Modification 2: Example of changing dot display format>

### (2-1: Transparency)

Although switching of display/hiding of dots has been described in the above embodiment, a display format of dots may be changed as described in Modification 2-1 below.

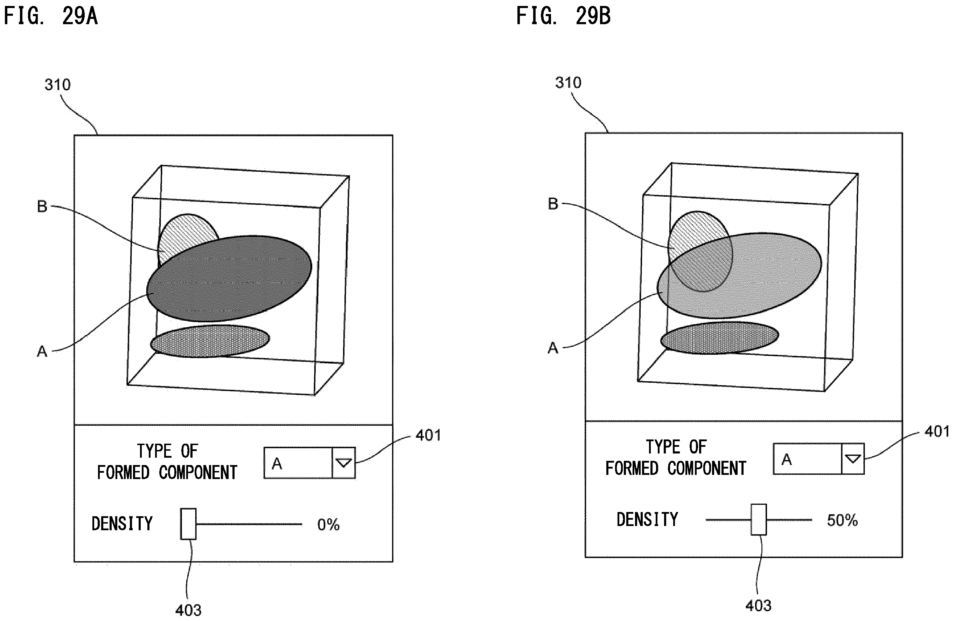

FIGS. 29A and 29B each schematically show a three-dimensional distribution chart 310 according to Modification 2-1.

In Modification 2-1, a slider bar 403 (hereinafter referred to as "bar") for changing transparency is displayed together with the three-dimensional distribution chart 310. In Modification 2-1, the transparency of dots can be continuously changed within a range of 0% to 100% by operating the display bar. In FIG. 29A, the transparency is set at 0%, and dots of a target formed component A is displayed in a default state. When the user changes the transparency to 50% by operating the bar, the dots of the target formed component A are displayed with the transparency of 50% as shown in FIG. 29B. Thus, dots of a formed component B having been hidden behind the formed component A emerge as shown in FIG. 29B.

The transparency being variable allows the user to observe a desired dot group through a dot group of another formed component the distribution of which overlaps the desired dot group on the three-dimensional distribution chart 310. As compared to the case where the other formed component overlapping the desired formed component is not displayed at all, distributions of the respective formed components can be easily compared and observed. Moreover, the transparency being continuously variable allows the user to display the dots with the density he/she desires. In observing a distribution chart, not only distribution of a specific cluster but also balance in distribution with another cluster may be observed in many cases. For example, there is a case where influence of spread of distribution of a cluster of lymphocytes on distribution of a cluster of monocytes is observed, and Modification 2-1 is applicable to such a case.

In the example shown in FIGS. 29A and 29B, the frequency of each dot may be changed as described with reference to FIGS. 28A and 28B, according to the change in the transparency. When the frequency of each dot is changed according to the transparency, another cluster that is hidden behind (i.e., that is present deeper in the depth direction of the displayed screen) can be observed more reliably. Moreover, at a coordinate at which two or more clusters compete, the dot color of a minority cluster emerges according to a reduction in transparency, whereby visibility of distribution of the hidden dot group is improved.

In this case, it is preferable not to hide the dots uniformly even when the recalculated dot frequency becomes less than 1. For example, even when the dot frequency of the formed component A at a certain coordinate becomes less than 1 because the transparency of the formed component A is reduced to 50%, the dots of the formed component A preferably remain displayed, unless another formed component whose frequency is not less than 1 is plotted at the same coordinate. In this case, denotation of the formed component remains visible even when the transparency is reduced, which is useful in comparing spreads of distributions of different types of clusters.

### (2-2: Designation of display order)

The dot display format may be changed not only by Modification 2-1 described above but also by Modification 2-2 below.

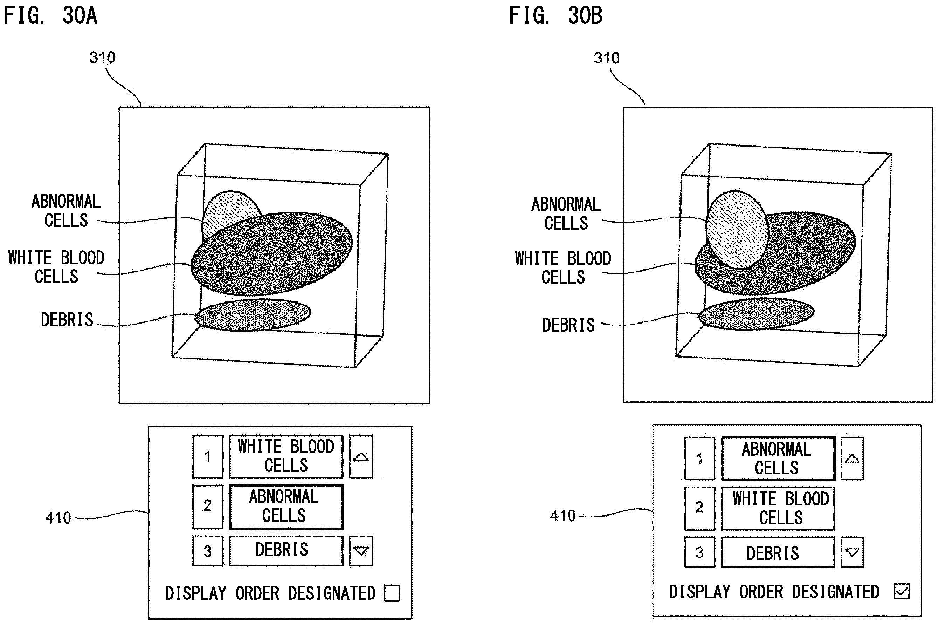

FIGS. 30A and 30B each schematically show a three-dimensional distribution chart 310 and a display order change area 410 according to Modification 2-2.

In Modification 2-2, the display order change area 410 for changing the dot display order is displayed on the screen 300 together with the three-dimensional distribution chart 310. The display order change area 410 includes a plurality of types of formed components to be displayed on the three-dimensional distribution chart 310, ranks in display order, a key for changing the display order, and a check box as to whether or not a display order is designated. In the display order change area 410, the plurality of types of formed components are displayed as a list.

The user can select one formed component from the display order change area 410. In the example of FIG. 30A, "abnormal cell" whose rank in the display order is "2" is selected. The user, with one type of formed component being selected, changes the display order by operating the key. The example of FIG. 30B shows a state where the rank of "abnormal cell" in the display order is changed from "2" to "1". Moreover, when designating the display order, the user can check the check box by clicking the same. The check box is unchecked in the default state. In this case, no display order is designated.

In the state where no display order is designated, a dot of a color according to the frequency of a formed component at each coordinate is rendered as described in the above embodiment. That is, when a plurality of types of formed components are plotted at the same coordinate, a dot of a color of a formed component type having the higher frequency is displayed while a dot of a color of a formed component type having the lower frequency is not displayed.

When the display order is changed and the check box is checked, the priority order of display of dots on the three-dimensional distribution chart 310 is changed according to the changed display order. Specifically, at a coordinate at which different types of formed components are plotted, a dot of a color of a formed component type whose rank in the display order is higher is preferentially rendered regardless of the frequency. In FIG. 30B, since the rank of abnormal cell is higher in the priority order of display than the rank of white blood cell, a dot of a color of abnormal cell is rendered while a color of white blood cell disappears at the coordinate at which both white blood cell and abnormal cell are plotted.

According to the change of the display order, even when dot groups of different types of formed components overlap each other, the user can observe a dot group of a desired type of formed component. Moreover, since dots of the other type of formed component are not hidden, the desired type of formed component can be observed in comparison with the other type of formed component.

In Modification 2-2, change in density as described in Modification 1 or change in transparency as described in Modification 2-1 may be additionally performed.

In Modifications 1 and 2, in response to the display format of specific dots being changed on the three-dimensional distribution chart 310, dots, on the two-dimensional distribution charts 321 to 323 or the surface plot charts 371 to 373, corresponding to the dots the display format of which has been changed on the three-dimensional distribution chart 310 may be displayed with the display format being changed.

### <Embodiment 2>

A specimen analysis device 1 according to Embodiment 2 is a device for analyzing urine as a specimen. The specimen analysis device 1 of Embodiment 2 has substantially the same configuration as the specimen analysis device 1 of Embodiment 1, but is different from the specimen analysis device 1 of Embodiment 1 in the following points.

In Embodiment 2, the sample preparation part 13 mixes a urine specimen with predetermined reagents to prepare an SF measurement sample in which red blood cells are not lysed, and a CR measurement sample in which red blood cells are lysed. Examples of formed components in the urine specimen include adipose particles, adipocyte, red blood cells, white blood cells, sperm, fungi, Trichomonas, epithelial cells, bacteria, casts, mucus fibers, and crystals. Examples of the urine specimen include discharged urine, and urine collected from an organism, such as primitive urine, urine in an ureter, urine in a bladder, and urine in an urethra.

The detector 14 (optical detector) has a configuration capable of receiving depolarized side scattered light. The detector 14 measures the SF measurement sample and the CR measurement sample on the basis of a flow cytometry method. The detector 14 applies light to each measurement sample that flows in a flow cell D1, receives forward scattered light, side scattered light, depolarized side scattered light, and side fluorescence generated from a formed component in the measurement sample, and outputs a detection signal having a waveform according to the intensity of each received light. A measurement channel based on the SF measurement sample is called "SF channel", and a measurement channel based on the CR measurement sample is called "CR channel".

The signal processing part 15 obtains measurement values of a plurality of parameters for each formed component, on the basis of the detection signals of the respective lights detected by the detector 14. For example, the measurement values of the parameters include: a forward scattered light intensity (FSC), a depolarized side scattered light intensity (DSS), and a side fluorescence intensity (SFL) which are obtained as waveform peak values; and a side fluorescence intensity (SFLH) detected with high sensitivity.

The controller 21, by using the measurement values based on the detector 14, classifies the formed components into a plurality of types, and obtains result values regarding the respective measurement items. The controller 21 performs classification and counting of red blood cells, casts, crystals, and the like. The controller 21 stores, in the storage 22, classification and measurement values of the respective formed components, and values regarding display/hiding corresponding to the classification values, as described in FIGS. 5 and 6.

Next, a display example of a screen displayed on the display 23 at step S17 of displaying the screen, according to Embodiment 2, will be described.

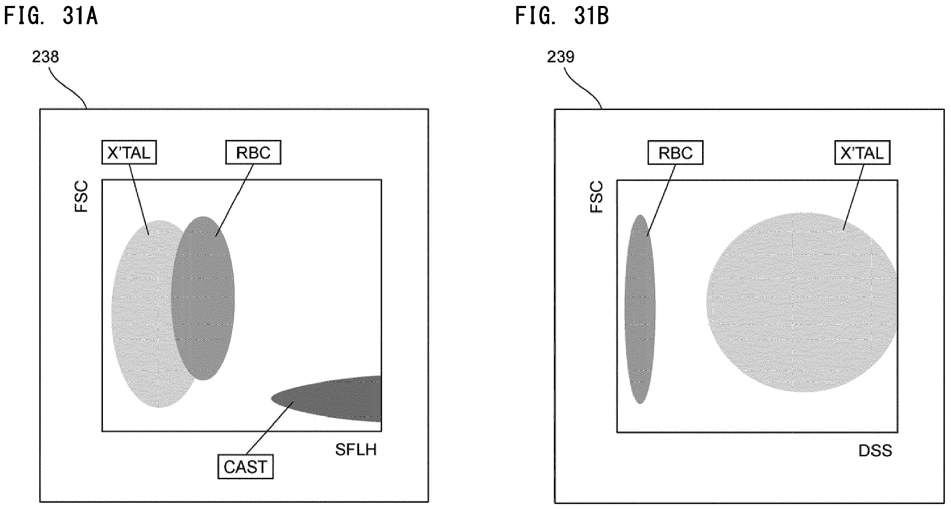

FIGS. 31A and 31B schematically show two-dimensional distribution charts 238 and 239 displayed in the graph display area 230 on a screen 200 similar to the screen 200 shown in FIG. 10. Each of the two-dimensional distribution charts 238 and 239 is displayed on the basis of classification and measurement values for each of formed components regarding the SF channel. In the two-dimensional distribution chart 238, the horizontal axis indicates SFLH and the vertical axis indicates FSC. In the two-dimensional distribution chart 239, the horizontal axis indicates DSS and the vertical axis indicates FSC. In each of the two-dimensional distribution charts 238 and 239, dot groups corresponding to the classified types are displayed in different colors.

In FIGS. 31A and 31B and FIG. 32 to FIG. 34B described below, clusters are given labels indicating the type names of the corresponding formed components in the three-dimensional distribution charts or the two-dimensional distribution charts, for convenience. Such a label including a character string of a type name may be given to any of the three-dimensional distribution charts, the two-dimensional distribution charts, and the surface plot charts of Embodiments 1 and 2. This allows the user to smoothly grasp the type names of the dot groups color-coded for each type.

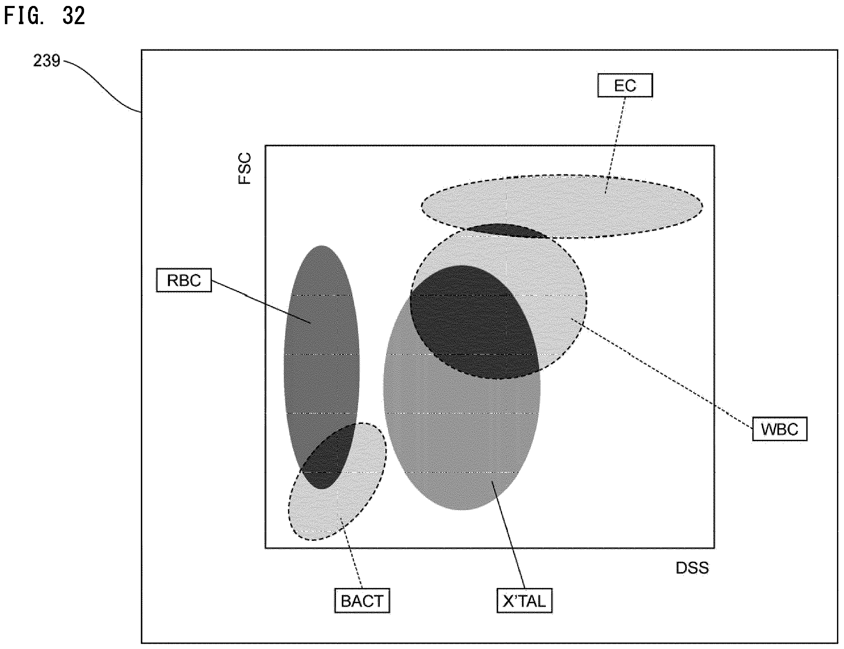

The user can grasp, to some extent, distribution of red blood cells (RBC) and crystals (X'TAL) by referring to the two-dimensional distribution chart 239. However, in actuality, as shown in FIG. 32, white blood cells (WBC), bacteria (BACT), and epithelial cells (EC) may be distributed so as to overlap the red blood cells (RBC) and the crystals (X'TAL). In this case, it is difficult to accurately grasp distribution of the red blood cells (RBC) and the crystals (X'TAL). Meanwhile, in Embodiment 2, since the three-dimensional distribution chart 310 including the three axes is displayed on the screen 300, the red blood cells and the crystals can be confirmed separately from the other formed components on the three-dimensional distribution chart 310.

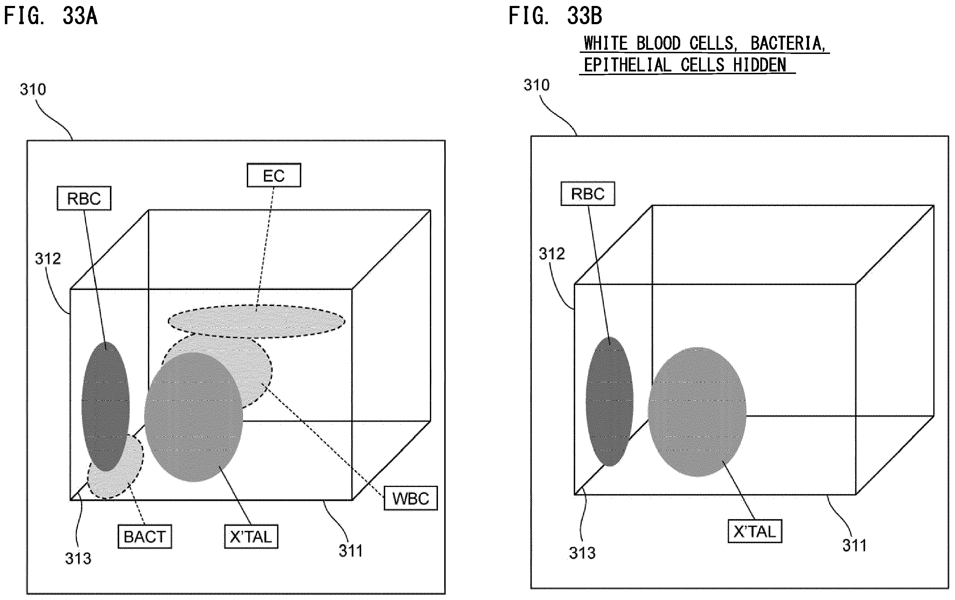

FIGS. 33A and 33B each schematically show a three-dimensional distribution chart 310 displayed on a screen 300 similar to the screen 300 shown in FIG. 12. In the three-dimensional distribution charts 310 shown in FIGS. 33A and 33B, an axis 311 indicates DSS, an axis 312 indicates FSC, and an axis 313 indicates SFL. The three-dimensional distribution charts 310 shown in FIGS. 33A and 33B are displayed on the basis of the two-dimensional distribution chart 238 shown in FIG. 31A or the two-dimensional distribution chart 239 shown in FIG. 31B. In each three-dimensional distribution chart 310, dot groups corresponding to the classified types are displayed in different colors.

In FIG. 33A, the SFL measurement values of white blood cells (WBC), bacteria (BACT), and epithelial cells (EC) are considerably larger than the SFL measurement values of red blood cells (RBC) and crystals (X'TAL). Therefore, in the three-dimensional distribution chart 310 shown in FIG. 33A, the red blood cells (RBC) and the crystals (X'TAL) are distributed separately, in the direction of the axis 313 (SFL), from the white blood cells (WBC), the bacteria (BACT), and the epithelial cells (EC). Therefore, the user can confirm the red blood cells and the crystals separately from the other formed components by changing the view angle.

When the three-dimensional distribution chart 310 shown in FIG. 33A is displayed, the user can hide the dot groups of white blood cells (WBC), bacteria (BACT), and epithelial cells (EC) as shown in FIG. 33B by performing an operation on the type selection area 330 in the screen 300. This allows the dot groups of red blood cells (RBC) and crystals (X'TAL) desired by the user to be more easily viewable on the three-dimensional distribution chart 310.

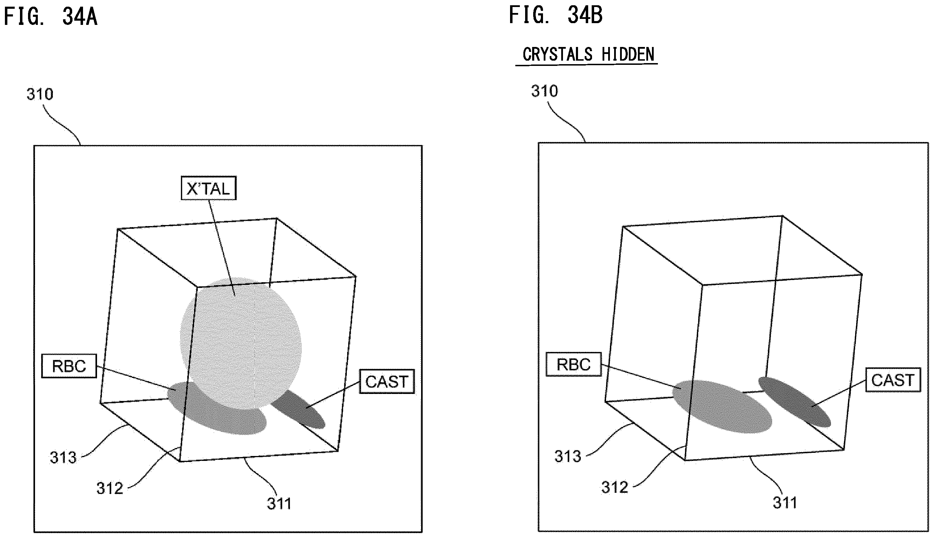

FIGS. 34A and 34B each schematically show a three-dimensional distribution chart 310 displayed on a screen 300 similar to the screen 300 shown in FIG. 12. In the three-dimensional distribution charts 310 shown in FIGS. 34A and 34B, an axis 311 indicates SFL, an axis 312 indicates DSS, and an axis 313 indicates FSC.

On the screen 300 shown in FIG. 33A, when the user performs an operation of hiding the dot groups of white blood cells (WBC), bacteria (BACT), and epithelial cells (EC), the screen 300 becomes the state of FIG. 34A, for example. In FIG. 34A, the dot group of crystals (X'TAL) overlaps the dot groups of red blood cells (RBC) and casts (CAST). From this state, the user performs an operation of hiding the dot group of crystal (X'TAL), whereby the screen 300 becomes the state shown in FIG. 34B. This allows the dot groups of red blood cells (RBC) and casts (CAST) desired by the user to be more easily visible.

### <Other modifications>

In the above embodiments, as shown in FIGS. 12 and 14, the two-dimensional distribution charts 321 to 323 or the surface plot charts 371 to 373 are displayed in the sub area 305 located at the right end of the screen 300. However, the sub area in which the two-dimensional distribution charts 321 to 323 or the surface plot charts 371 to 373 are displayed may be any area as long as it is displayed simultaneously with the screen 300 on the display 23, and may be a sub area in another screen that is displayed simultaneously with the screen 300.

In the above embodiments, a label indicating a parameter name may be imparted to each of the axes 311 to 313 of the three-dimensional distribution chart 310. Moreover, a label indicating a parameter name may be imparted to each of the axes of the two-dimensional distribution charts 321 to 323 and the surface plot charts 371 to 373. These labels allow the user to smoothly grasp the parameter names corresponding to the respective axes.

In the above embodiments, the three-dimensional distribution chart 310 is displayed after a predetermined operation has been performed by the user through any of the two-dimensional distribution charts 231 to 237. However, the screen 300 including the three-dimensional distribution chart 310 may be displayed after a predetermined operation has been performed by the user not through any of the two-dimensional distribution charts 231 to 237.

In the above embodiments, the three parameters set in the three-dimensional distribution chart 310 may not necessarily be the measurement values such as a peak value and a width of a waveform of a detection signal from the detector 14. For example, one parameter out of the three parameters may be a value obtained through calculation performed on the measurement values of the remaining two parameters. Alternatively, two parameters out of the three parameters may be values obtained through calculation performed on the measurement value of the remaining one parameter. Still alternatively, one or more parameters out of the three parameters may be values obtained through calculation or analysis performed on the detection signals from the detector 14.

In the above embodiments, formed components are classified on the basis of the measurement values of a plurality of parameters obtained from the formed components. However, the classification method is not limited thereto. The type of a formed component may be identified by analyzing, with a deep learning algorithm, waveform data reflecting morphological features of the formed component. For example, an analog detection signal having a waveform corresponding to light (scattered light and fluorescence) generated from each formed component that flows in the flow cell D1 is obtained from the formed component, and the analog detection signal is sampled at a predetermined rate (e.g., 1024 points at a 10 nanosecond interval), thereby obtaining digital waveform data reflecting the morphological features of the formed component. The waveform data thus obtained is inputted to a deep learning algorithm having a neural network structure trained by using, as teacher data, waveform data of formed components whose types have already been known, thereby determining the type of the formed component corresponding to the waveform data. Such a classification method is disclosed in WO2020/196074, for example. WO2020/196074 and Japanese Laid-Open Patent Publication based thereon are incorporated herein by reference.

Various modifications of the embodiments of the present invention may be made as appropriate without departing from the scope of the technical idea defined by the claims.

For example, the present invention provides the following items (1) to (33).
(1) A method for displaying a result of analysis performed on a specimen containing formed components, the method comprising:
   obtaining, from each of the formed components, measurement values of at least three parameters;
   displaying a screen including a three-dimensional distribution chart in which the three parameters are used as axes and the formed components are represented by dots; and
   hiding specific dots on the three-dimensional distribution chart or changing a display format of the specific dots, according to an operation performed by a user.
(2) The display method of item (1), further comprising
   classifying the formed components on the basis of the measurement values or waveform data reflecting morphological features of the formed components.
(3) The display method of item (2), wherein
   the hiding or changing includes:
   receiving selection of types of formed components from among the classified formed components; and
   for each of the types of the formed components having been selected, hiding the specific dots or chancing the display format of the specific dots.
(4) The display method of any one of items (1) to (3), further comprising
   changing a view angle of the three-dimensional distribution chart according to an operation performed on the three-dimensional distribution chart in a state where the specific dots are hidden or are displayed with the display format changed.
(5) The display method of item (2) or (3), wherein
   the displaying the screen including the three-dimensional distribution chart, includes
   color-coding the dots for each type and displaying the dots.
(6) The display method of any one of items (1) to (5), wherein
   the hiding or changing includes:
   receiving selection of the specific dots to be displayed on the three-dimensional distribution chart with the display format changed; and
   receiving an operation of changing the display format of the specific dots having been selected.
(7) The display method of item (6), wherein
   the hiding or changing includes
   changing a priority order of display of the specific dots corresponding to the type of the formed component having been selected.
(8) The display method of any one of items (1) to (7), further comprising:
   displaying a plurality of two-dimensional distribution charts having different measurement channels; and
   receiving selection of any of the plurality of two-dimensional distribution charts, wherein
   the three-dimensional distribution chart to be displayed in the displaying the screen including the three-dimensional distribution chart is a three-dimensional distribution chart corresponding to the measurement channel of the selected two-dimensional distribution chart.
(9) The display method of any one of items (1) to (8), wherein
   the displaying the screen including the three-dimensional distribution chart, includes
   selecting three axes of the three-dimensional distribution chart from four or more parameters.
(10) The display method of any one of items (1) to (9), wherein
   three axes used in the three-dimensional distribution chart are color-coded, and
   a color of each axis and a parameter name set to each axis are displayed.
(11) The display method of any one of items (1) to (10), wherein
   the displaying the screen including the three-dimensional distribution chart, includes
   setting a display condition for the three-dimensional distribution chart for each of specimen measurement modes.
(12) The display method of any one of items (1) to (11), wherein
   the displaying the screen including the three-dimensional distribution chart, includes
   setting a display condition for the three-dimensional distribution chart, for each of measurement channels to be used in the obtaining the measurement values.
(13) The display method of any one of items (1) to (12), wherein
   the displaying the screen including the three-dimensional distribution chart, includes
   displaying a two-dimensional distribution chart or a surface plot chart corresponding to the three-dimensional distribution chart in an area, in the screen, different from an area where the three-dimensional distribution chart is displayed.
(14) The display method of item (13), wherein
   the displaying the screen including the three-dimensional distribution chart, includes
   setting two parameters, out of the three parameters used as three axes of the three-dimensional distribution chart, as parameters for the two-dimensional distribution chart or the surface plot chart.
(15) The display method of item (14), wherein
   the displaying the screen including the three-dimensional distribution chart, includes:
   receiving setting of the parameters to be used as the axes of the three-dimensional distribution chart; and
   changing the parameters for the two-dimensional distribution chart or the surface plot chart on the basis of the parameters having been received.
(16) The display method of any one of items (13) to (15), wherein
   the displaying the screen including the three-dimensional distribution chart is performed, in response to hiding the specific dots or changing the display format, such that dots, on the two-dimensional distribution chart or the surface plot chart, which correspond to the specific dots that are hidden or are changed in the display format are hidden or are displayed with a display format changed.
(17) The display method of any one of items (13) to (16), wherein
   the axes used in the three-dimensional distribution chart and the two-dimensional distribution chart or the surface plot chart are color-coded such that the axes of the same parameter are given the same color between the three-dimensional distribution chart, and the two-dimensional distribution chart or the surface plot chart.
(18) The display method of any one of items (1) to (17), further comprising
   displaying reference areas corresponding to clusters of the formed components so as to overlap the respective clusters on the three-dimensional distribution chart.
(19) The display method of any one of items (1) to (18), further comprising
   switching the three-dimensional distribution chart to the three-dimensional distribution chart of another specimen in response to a specimen switching operation.
(20) The display method of item (19), wherein
   the switching to the three-dimensional distribution chart of said another specimen, includes
   displaying the three-dimensional distribution chart of the other specimen, with a display condition before the switching being maintained.
(21) The display method of any one of items (1) to (20), further comprising
   storing, for each formed component, the measurement value, information for identifying the dots, and information regarding display/hiding of the specific dots in association with each other, wherein
   the hiding or changing includes
   hiding the specific dots or changing the display format of the specific dots, on the basis of the stored information regarding display/hiding of the specific dots.
(22) A specimen analysis device for analyzing a specimen containing formed components, comprising:
   a detector configured to obtain a detection signal from each formed component;
   a signal processing part configured to obtain measurement values of at least three parameters from the detection signal;
   a display configured to display a screen including a three-dimensional distribution chart in which the three parameters are used as axes and the formed components are represented by dots; and
   a controller configured to control, according to an operation performed by a user, the display to hide specific dots on the three-dimensional distribution chart or change a display format of the specific dots.
(23) The specimen analysis device of item (22), wherein
   the controller is configured to classify the formed components on the basis of the measurement values.
(24) The specimen analysis device of item (23), further comprising an input part, wherein
   the controller receives, through the input part, selection of types of formed components from among the classified formed components, and controls the display to hide the specific dots or change a display format of the specific dots, for each of the types of the formed components having been selected.
(25) The specimen analysis device of item (24), wherein
   the controller receives, through the input part, selection of the dots to be displayed on the three-dimensional distribution chart, and controls the display to display the dots having been selected on the three-dimensional distribution chart.
(26) The specimen analysis device of item (24) or (25), wherein
   the controller receives, through the input part, selection of the specific dots to be hidden on the three-dimensional distribution chart, and controls the display to hide the specific dots having been selected on the three-dimensional distribution chart.
(27) The specimen analysis device of any one of items (24) to (26), wherein
   the controller receives, through the input part, selection of the specific dots to be displayed on the three-dimensional distribution chart with the display format changed, and controls the display to display the specific dots on the three-dimensional distribution chart with the display format changed, in response to an operation of changing the display format of the specific dots having been selected.
(28) The specimen analysis device of any one of items (22) to (27), further comprising
   a storage configured to store, for each formed component, the measurement values, information for identifying the dots, and information regarding display/hiding of the specific dots in association with each other, wherein
   the controller controls the display to hide the specific dots on the three-dimensional distribution chart or change the display format of the specific dots, on the basis of the information regarding display/hiding of the specific dots and stored in the storage.
(29) The specimen analysis device of any one of items (22) to (28), wherein
   the detector includes an optical detector configured to apply light to a flow cell in which a measurement sample prepared by mixing the specimen with a reagent flows, and receive light generated from a formed component in the measurement sample to obtain a detection signal from the formed component.
(30) A method for displaying a result of analysis performed on a specimen containing formed components, the method comprising:
   obtaining measurement values from each of the formed components;
   obtaining a data set including at least three parameters based on the measurement values, and an attribute of the formed component;
   displaying a screen including a three-dimensional distribution chart in which the formed component is represented by dots, on the basis of the data set; and
   displaying/hiding the dots on the three-dimensional distribution chart or changing a display format of the dots, on the basis of the attribute.
(31) The display method of item (30), further comprising
   classifying the formed components on the basis of the measurement values or waveform data reflecting morphological features of the formed components.
(32) The display method of item (31), wherein
   the displaying/hiding or changing includes:
   receiving selection of types of formed components from among the classified formed components; and
   displaying/hiding the dots or changing the display format of the dots, for each of the types of the formed components having been selected.
(33) The display method of any one of items (30) to (32), further comprising
   changing a view angle of the three-dimensional distribution chart, according to an operation performed on the three-dimensional distribution chart in a state where the dots are hidden or are displayed with the display format changed.

### DESCRIPTION OF THE REFERENCE CHARACTERS

- 1: specimen analysis device
- 14: detector
- 15: signal processing part
- 21: controller
- 22: storage
- 23: display
- 24: input part
- 231 to 237: two-dimensional distribution chart
- 300: screen
- 310: three-dimensional distribution chart
- 311 to 313: axis
- 314a to 314d: reference area
- 321 to 323: two-dimensional distribution chart
- 381 to 383: surface plot chart
- D1: flow cell

## Claims

1. A method for displaying a result of analysis performed on a specimen containing formed components, the method comprising:
obtaining, from each of the formed components, measurement values of at least three parameters;
displaying a screen including a three-dimensional distribution chart in which the three parameters are used as axes and the formed components are represented by dots; and
hiding specific dots on the three-dimensional distribution chart or changing a display format of the specific dots, according to an operation performed by a user.

2. The display method of claim 1, further comprising
classifying the formed components on the basis of the measurement values or waveform data reflecting morphological features of the formed components.

3. The display method of claim 2, wherein
the hiding or changing includes:
receiving selection of types of formed components from among the classified formed components; and
for each of the types of the formed components having been selected, hiding the specific dots or changing the display format of the specific dots.

4. The display method of any one of claims 1 to 3, further comprising
changing a view angle of the three-dimensional distribution chart according to an operation performed on the three-dimensional distribution chart in a state where the specific dots are hidden or are displayed with the display format changed.

5. The display method of claim 2 or 3, wherein
the displaying the screen including the three-dimensional distribution chart, includes
color-coding the dots for each type and displaying the dots.

6. The display method of any one of claims 1 to 5, wherein
the hiding or changing includes:
receiving selection of the specific dots to be displayed on the three-dimensional distribution chart with the display format changed; and
receiving an operation of changing the display format of the specific dots having been selected.

7. The display method of claim 6, wherein
the hiding or changing includes
changing a priority order of display of the specific dots corresponding to the type of the formed component having been selected.

8. The display method of any one of claims 1 to 7, further comprising:
displaying a plurality of two-dimensional distribution charts having different measurement channels; and
receiving selection of any of the plurality of two-dimensional distribution charts, wherein
the three-dimensional distribution chart to be displayed in the displaying the screen including the three-dimensional distribution chart is a three-dimensional distribution chart corresponding to the measurement channel of the selected two-dimensional distribution chart.

9. The display method of any one of claims 1 to 8, wherein
the displaying the screen including the three-dimensional distribution chart, includes
selecting three axes of the three-dimensional distribution chart from four or more parameters.

10. The display method of any one of claims 1 to 9, wherein
three axes used in the three-dimensional distribution chart are color-coded, and
a color of each axis and a parameter name set to each axis are displayed.

11. The display method of any one of claims 1 to 10, wherein
the displaying the screen including the three-dimensional distribution chart, includes
setting a display condition for the three-dimensional distribution chart for each of specimen measurement modes.

12. The display method of any one of claims 1 to 11, wherein
the displaying the screen including the three-dimensional distribution chart, includes
setting a display condition for the three-dimensional distribution chart, for each of measurement channels to be used in the obtaining the measurement values.

13. The display method of any one of claims 1 to 12, wherein
the displaying the screen including the three-dimensional distribution chart, includes
displaying a two-dimensional distribution chart or a surface plot chart corresponding to the three-dimensional distribution chart in an area, in the screen, different from an area where the three-dimensional distribution chart is displayed.

14. The display method of claim 13, wherein
the displaying the screen including the three-dimensional distribution chart, includes
setting two parameters, out of the three parameters used as three axes of the three-dimensional distribution chart, as parameters for the two-dimensional distribution chart or the surface plot chart.

15. The display method of claim 14, wherein
the displaying the screen including the three-dimensional distribution chart, includes:
receiving setting of the parameters to be used as the axes of the three-dimensional distribution chart; and
changing the parameters for the two-dimensional distribution chart or the surface plot chart on the basis of the parameters having been received.
